(19) Europäisches Patentamt
European Patent Office
Office européen des brevets

(11) **EP 4 456 075 A1**

(12) **EUROPEAN PATENT APPLICATION**
published in accordance with Art. 153(4) EPC

(43) Date of publication:
**30.10.2024 Bulletin 2024/44**

(51) International Patent Classification (IPC):
**G16B 25/10** (2019.01)    **G16B 50/10** (2019.01)
**G16B 20/20** (2019.01)    **G16B 45/00** (2019.01)

(21) Application number: **22911799.9**

(52) Cooperative Patent Classification (CPC):
**G16B 25/10; G16B 20/00; G16B 20/20;**
**G16B 25/00; G16B 40/00; G16B 40/20;**
**G16B 45/00; G16B 50/00; G16B 50/10;**
**G16H 50/30**

(22) Date of filing: **19.12.2022**

(86) International application number:
**PCT/KR2022/020687**

(87) International publication number:
**WO 2023/121166 (29.06.2023 Gazette 2023/26)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **20.12.2021 KR 20210183137**

(71) Applicant: **IUCF-HYU (INDUSTRY-UNIVERSITY**
**COOPERATION**
**FOUNDATION HANYANG UNIVERSITY)**
**Seoul 04763 (KR)**

(72) Inventors:
• **KIM, Jin Hyuk**
**Seongnam-si, Gyeonggi-do 13589 (KR)**
• **KIM, Hye Young**
**Seongnam-si, Gyeonggi-do 13597 (KR)**

(74) Representative: **dompatent von Kreisler Selting**
**Werner -**
**Partnerschaft von Patent- und Rechtsanwälten**
**mbB**
**Deichmannhaus am Dom**
**Bahnhofsvorplatz 1**
**50667 Köln (DE)**

(54) **GENE ONTOLOGY-BASED GENETIC DATA ANALYSIS METHOD AND ANALYSIS DEVICE**

(57)    A method of analyzing genetic data based on gene ontology includes receiving, by an analysis device, gene expression data of a sample and gene expression data of reference tissue, receiving, by the analysis device, information on gene set related to at least one gene ontology term, extracting, by the analysis device, first gene expression data for genes belonging to the gene set from among the gene expression data of the sample and second gene expression data for genes belonging to the gene set from among the gene expression data of the reference tissue, and calculating, by the analysis device, a degree of variation of the sample based on the reference tissue by calculating entropy for the first gene expression data and the second gene expression data.

EP 4 456 075 A1

**FIG. 1**

**Description**

[Technical Field]

The following description relate to a technique for analyzing genetic data of a sample based on expression information of genes calculated through gene ontology.

[Background Art]

Traditionally, the cause of diseases such as malignant tumors is assumed to be in the genome. Therefore, research to overcome malignant tumors is focused on the genome. The development of molecular biology has made it possible to use molecularly targeted treatment that reduces the side effects of traditional cancer chemotherapy and selectively destroys only cancer cells. However, perfect treatment for malignant tumors is still impossible. This results from a lack of understanding of the functions and mechanisms of the genome. Traditional research methods for the genome are based on biochemical techniques and have limitations in expanding understanding beyond chemical functions.

[Disclosure]

[Technical Problem]

The following description provide a technique for analyzing a sample based on transcriptional expression information of genes in a sample. The following description provides a technique for analyzing a sample by interpreting gene expression information in a gene space for genes determined based on gene ontology.

[Technical Solution]

In one general aspect, there is provided a method of analyzing genetic data based on gene ontology includes receiving, by an analysis device, gene expression data of a sample and gene expression data of reference tissue, receiving, by the analysis device, information on gene set related to at least one gene ontology term, extracting, by the analysis device, first gene expression data for genes belonging to the gene set from among the gene expression data of the sample and second gene expression data for genes belonging to the gene set from among the gene expression data of the reference tissue, and calculating, by the analysis device, a degree of variation of the sample based on the reference tissue by calculating entropy for the first gene expression data and the second gene expression data.

In another aspect, there is provided an device for analyzing genetic data based on gene ontology includes an input device that receives information on gene set related to at least one gene ontology term, a storage device that stores gene expression data of a sample and gene expression data of reference tissue, and a calculation device that extracts first gene expression data for genes belonging to the gene set from among the gene expression data of the sample and second gene expression data for genes belonging to the gene set from among the gene expression data of the reference tissue, and calculates a degree of variation of the sample based on the reference tissue by calculating entropy for the first gene expression data and the second gene expression data.

[Advantageous Effects]

The following description is a new approach to analyze effectively a phenotype of a sample using only gene expression information. The following description provides a diagnosis and treatment for a specific disease by interpreting conventional gene ontology information into information for treatment the disease.

[Brief description of Drawings]

FIG. 1 is an example of a system for analyzing a sample based on gene ontology.
FIG. 2A is an example showing a transcriptional state of a single gene.
FIG. 2B is an example of a transcriptional state vector of the genes in FIG. 2A.
FIG. 3A is an example illustrating a transcriptional state of two genes.
FIG. 3B is the result of projecting a density matrix onto a plane composed of basis transcriptional states with high dwelling probability in FIG. 3A.

FIG. 4A is an example illustrating a transcriptional state of three genes.

FIG. 4B is an example of a histogram for the dwelling probability of the basis transcriptional state in FIG. 4A.

FIG. 4C is a diagram illustrating the results of projecting the density matrix onto the plane composed of the basis transcriptional states with high dwelling probability in FIG. 3A.

FIG. 5 is a diagram illustrating the results of performing a one-way ANOVA test on the entropy of a cell cycle-related gene ontology (GO) term gene set.

FIG. 6 is an example of separately analyzing the correlations between the GO term entropy of normal breast tissue (BRCA) and the GO term entropy of BRNO according to a sample probability of BRCA.

FIG. 7 is an example of analyzing the correlations between the GO term entropy of COAD and the GO term entropies of rectal cancer (READ), colon cancer (COAD), BRCA, and lung squamous cell carcinoma (LUSC).

FIG. 8 is an example of the relative entropy of tissue y with respect to tissue x.

FIG. 9 is an example of analyzing the correlation between the GO term relative entropy of each tissue with respect to BRNO.

FIG. 10 is an example of analyzing the correlation between the GO term angular divergence (AD) of each tissue with respect to BRNO.

FIG. 11 is an example of separately analyzing the correlations between the GO term relative entropy and AD of BRCA and the GO term relative entropy and AD of LUSC with respect to BRNO according to the sample probability of BRCA.

FIG. 12 is an example of comparing the GO term relative entropy of each tissue with respect to BRNO.

FIG. 13 is an example of comparing the GO term AD of each tissue with respect to BRNO.

FIG. 14 is an example of displaying the sample probability of a gene space of a genomic system having a density matrix p.

FIG. 15 is an example of calculating the GO sample probability (GSP) of BRNO and BRCA samples for the density matrix of BRNO and the GSP of the BRNO and BRCA samples for the density matrix of BRCA in 78 cell cycle-related GO terms.

FIG. 16 is an example of a GSP distribution of normal tissue samples and the GSP distribution of tumor samples for density matrices of normal genomes and tumor genomes.

FIG. 17 is an example of performing hierarchical clustering of BRCA samples based on the GSP for specific GO terms.

FIG. 18 is an example of describing a log odds ratio (LOR) of a specific gene in an individual sample for a genomic system having a density matrix p.

FIG. 19 is an example of comparing the GO log odds ratio (GLOR) of each gene with the log expression ratio (LER) for a genomic system of a specific GO term in BRCA and BRNO samples.

FIG. 20 is an example of the GLOR and LER of the abnormal spindle-like microcephaly-associated (ASPM) gene for a genomic system of cell cycle-related GO term Nos. 6 and 46 in BRCA and BRNO samples.

FIG. 21 is an example of a process of calculating an analysis index for a gene ontology-based sample.

FIG. 22 is another example of the process of calculating an analysis index for a gene ontology-based sample.

FIG. 23 is an example of an analysis device that analyzes gene ontology-based gene data.

[Exemplary embodiments of invention]

The technology described below may be variously modified and have several embodiments. Therefore, specific embodiments will be illustrated in the accompanying drawings and described in detail. However, it is to be understood that the technology described below is not limited to specific embodiments, but includes all modifications, equivalents, and substitutions included the scope and spirit of the technology described below.

Terms such as "first," "second," "A," "B," and the like, may be used to describe various components, but the components are not limited by the terms, and are used only for distinguishing one component from other components. For example, a first component may be named a second component and the second component may also be named the first component without departing from the scope of the technology described below. The term and/or includes a combination of a plurality of related described items or any one of the plurality of related described items.

It should be understood that singular expressions include plural expressions unless the context clearly indicates otherwise, and it will be further understood that the terms "comprise" and "have" used in this specification specify the presence of stated features, steps, operations, components, parts, or a combination thereof, but do not preclude the presence or addition of one or more other features, steps, operations, components, parts, or a combination thereof.

Prior to the detailed description of the drawings, it is intended to clarify that the components in this specification are merely distinguished by the main functions of each component. That is, two or more components to be described below may be combined into one component, or one component may be divided into two or more components for more detailed functions. In addition, each of the constituent parts to be described below may additionally perform some or all of the functions of other constituent parts in addition to the main functions of the constituent parts, and some of the main functions of the constituent parts may be performed exclusively by other components.

In addition, in performing the method or the operation method, each of the processes constituting the method may occur in a different order from the specified order unless a specific order is explicitly described in the context. That is, each process may be performed in the same order as specified, performed at substantially the same time, or performed in the opposite order.

Terms used in the following description will be described first.

A sample may basically mean a living entity. The entity basically includes humans, animals, plants, microorganisms, etc. However, in the following description, it is assumed that the samples are from humans. The sample may be represented by a sample acquired from a subject to be analyzed. Accordingly, the sample includes meanings such as an individual, individual's tissue, an individual's cell set, etc.

Sample data refers to gene expression data of the sample.

Gene expression means that a gene is transcribed into a ribonucleic acid (RNA) product.

The gene expression data is a data set that shows the expression level of genes. Meanwhile, the gene expression data may be calculated using techniques such as a microarray and next generation sequencing (NGS).

Gene ontology (GO) corresponds to a computerized data model that analyzes the attributes of genes and gene products of living things (including humans). The GO provides classification of gene-related information. The GO is constructed in the form of a public database. The GO is generally defined at independent levels (sub-ontologies) such as molecular function, biological process, and cellular component. For example, cytochrome c, which is a gene product, may be described as oxidoreductase activity in terms of molecular function, oxidative phosphorylation in terms of biological process, and mitochondrial matrix in terms of cellular component.

Gene ontology terms (GO terms) are a way of expressing genes and gene products using GO. GO terms include identifiers, definitions, the above-described sub-ontologies, and relationships with other terms. The GO may also be expressed in the form of a graph with GO terms as nodes. That is, a gene or gene product is expressed in terms of association with other objects.

Entropy indicates the functional unity and characteristic activity of a plurality of genes.

Sample probability (hereinafter referred to as SP) refers to the probability of each sample for genes belonging to a certain gene group. For example, SP may correspond to a quantified value representing the degree of variation of a specific sample based on normal tissue for all genes in a sample.

A log odds ratio (hereinafter referred to as LOR) is a log value for a ratio of a first probability when the gene is in the gene group and a second probability when the gene is not in the gene group, based on a specific gene. The LOR may indicate the effect of a specific gene on a genomic system.

Hereinafter, GO-based sample (genetic data) analysis may be performed in a certain analysis device. The analysis device refers to a computer device that can process and compute input data. For example, the analysis device may be any one of devices such as a personal computer (PC), a smartphone, a server, and a chipset in which a program is embedded.

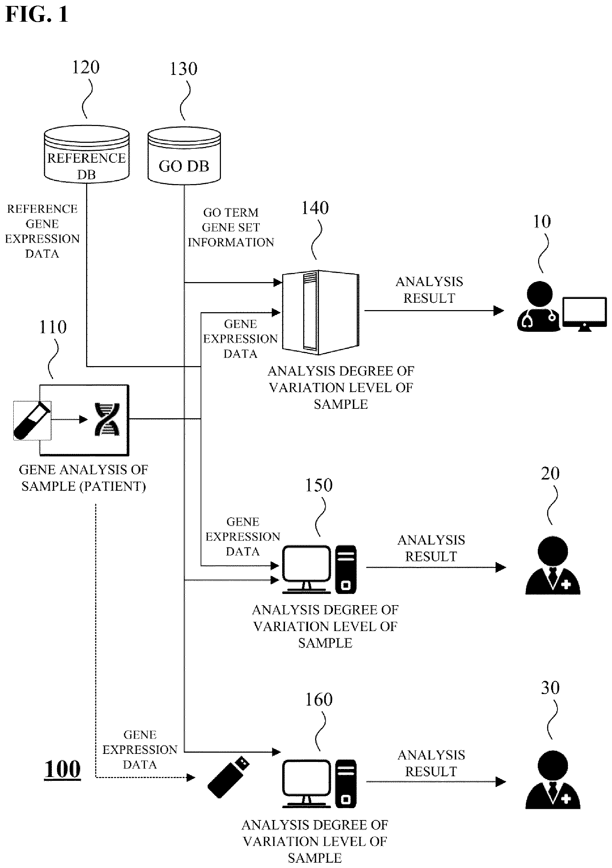

FIG. 1 is an example of a system 100 for analyzing a sample based on gene ontology. In FIG. 1, analysis devices 140, 150, and 160 analyze the genetic data of a sample. In FIG. 1, the analysis device is shown in the form of a server 140 and computer terminals 150 and 160.

A genome analysis device 110 generates gene expression data by analyzing samples from patients with specific diseases. Genetic data may be data about deoxyribo nucleic acid (DNA) or RNA sequences. The genome analysis device 110 produces gene expression data in the form of digital data.

A reference database 120 stores reference gene expression data for a specific disease. The reference gene expression data may be gene expression data of normal people.

A GO database 130 stores GO information. The GO database 130 may output information on gene(s) related to a specific GO term.

The server 140 receives the gene expression data for the sample from the genome analysis device 110. The server 140 receives the reference gene expression data from the reference database 120.

The server 140 may receive genetic information related to a specific GO term from the GO database 130. Here, the specific GO term may be a single term or a plurality of terms. The genetic information related to the specific GO term is information on a gene set related to the corresponding GO term and may include identification information of genes included in the corresponding gene set.

The server 140 may query the GO database 130 for a specific GO term and receive the genetic information related to the GO term. Alternatively, a separate computer device may query the GO database 130 to transmit the genetic information related to the GO term to the server 140.

The server 140 extracts only information on a gene set related to the GO term from the gene expression data for the sample. In addition, the server 140 extracts only the information on the gene set related to the GO term from the reference gene expression data. The server 140 may calculate an index indicating the degree of variation of the sample using the expression data of the gene set of the sample data and the expression data of the gene set of the reference data. A user 10 may access the server 140 through a user terminal (PC, smart phone, etc.) and check the analysis results performed by the server 140.

The computer terminal 150 receives the gene expression data for the sample from the genome analysis device 110. The computer terminal 150 receives the reference gene expression data from the reference database 120.

The computer terminal 150 may receive the genetic information related to a specific GO term from the GO database 130. Here, the specific GO term may be a single term or a plurality of terms. The genetic information related to the specific GO term is information on the gene set related to the corresponding GO term and may include the identification information of genes included in the corresponding gene set.

The computer terminal 150 may query the GO database 130 for a specific GO term and receive the genetic information related to the GO term. Alternatively, a separate computer device may query the GO database 130 to transmit the genetic information related to the GO term to the computer terminal 150.

The computer terminal 150 extracts only the information on the gene set related to the GO term from the gene expression data for the sample. In addition, the computer terminal 150 extracts only the information on the gene set related to the GO term from the gene expression data. The computer terminal 150 may calculate an index indicating the degree of variation of the sample using the expression data of the gene set of the sample data and the expression data of the gene set of the reference data. A user 20 may check the analysis results through the computer terminal 150 used by the user 20.

The computer terminal 160 receives genetic data through a medium (e.g., universal serial bus (USB), secure digital (SD) card, etc.) in which the gene expression data for the sample generated by the genome analysis device 110 is stored.

The computer terminal 160 receives the gene expression data for the sample from the genome analysis device 110. The computer terminal 160 receives the reference gene expression data from the reference database 120.

The computer terminal 160 may receive the genetic information related to a specific GO term from the GO database 130. Here, the specific GO term may be a single term or a plurality of terms. The genetic information related to the specific GO term is information on the gene set related to the corresponding GO term, and may include the identification information of genes included in the corresponding gene set.

The computer terminal 160 may query the GO database 130 for a specific GO term and receive the genetic information related to the GO term. Alternatively, a separate computer device may query the GO database 130 to transmit the genetic information related to the GO term to the computer terminal 160.

The computer terminal 160 extracts only the information on the gene set related to the GO term from the gene expression data for the sample. In addition, the computer terminal 160 extracts only the information on the gene set related to the GO term from the gene expression data. The computer terminal 160 may calculate the index indicating the degree of variation of the sample using the expression data of the gene set of the sample data and the expression data of the gene set of the reference data. A user 30 may confirm the analysis results through the computer terminal 160 used by the user 30.

Hereinafter, the process and indexes by which the analysis device analyzes the gene set extracted by GO will be described in detail.

First, a genomic state defined by researchers will be described.

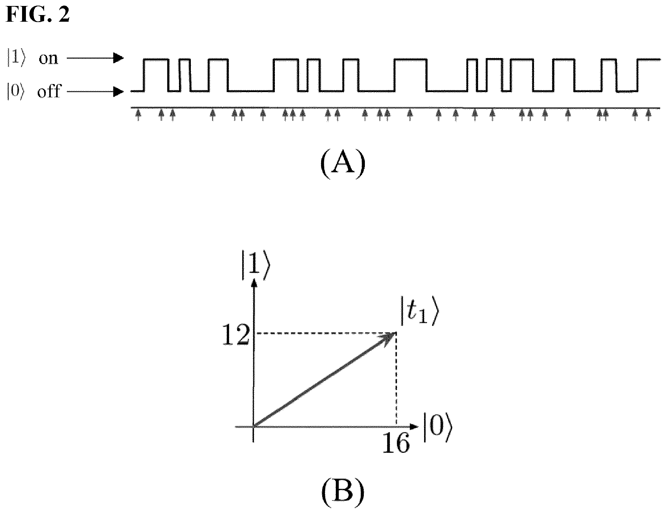

Assuming a genome including only one gene, the gene may be divided into an active state "on," which receives transcriptional stimulation, and an inactive state "off," which does not receive stimulation. FIG. 2A is an example showing a transcriptional state of a single gene. A transcriptional state $t_1$ of a single gene may be defined as a basis transcriptional state at multiple time points. In FIG. 2A, the arrow indicates the time point when the basis transcriptional state is measured. In FIG. 2A, the number of off states and the number of on states of the corresponding gene are 16 and 12, respectively.

The transcriptional states "off" and "on" are mutually exclusive. The "off" and "on" may be expressed as being geometrically orthonormal. Therefore, the "off" state may be expressed as $|0\rangle$, and the "on" state may be expressed as $|1\rangle$. $\langle 0|1\rangle$ is 0. FIG. 2B is an example of a transcriptional state vector of the genes in FIG. 2A. In geometric terms, a transcriptional state vector $|t_1\rangle$ of the gene in FIG. 2A is $16|0\rangle + 12|1\rangle$. Therefore, the dwelling probabilities of each basis transcriptional state using the Pythagorean theorem are $p_{off} = 16^2/(16^2 + 12^2)$ and $p_{on} = 12^2/(16^2 + 12^2)$. To generalize this further, the dwelling probability is a diagonal of dyad $|t_1\rangle\langle t_1|$ of $t_1$ with a trace normalized to 1, and is as shown in Equation 1 below.

[Equation 1]

$$\rho = \frac{|t_1\rangle\langle t_1|}{\mathrm{Tr}(|t_1\rangle\langle t_1|)} = \frac{\begin{bmatrix}16\\12\end{bmatrix}\begin{bmatrix}16 & 12\end{bmatrix}}{\mathrm{Tr}\left(\begin{bmatrix}16\\12\end{bmatrix}\begin{bmatrix}16 & 12\end{bmatrix}\right)} = \begin{bmatrix}0.64 & 0.48\\0.48 & 0.36\end{bmatrix}$$

Here, ρ is a density matrix of the transcriptional state $t_1$. ρ is equal to $\rho^2$ and thus is a pure state. In addition, the diagonal of ρ is the dwelling probability of $|0\rangle$ and $|1\rangle$ in that order.

Furthermore, in a genome composed of two genes, the transcriptional state may be expressed as follows. Assuming a genome composed of gene 1 and gene 2, the transcriptional state of each gene is referred to as $t_1$ and $t_2$.

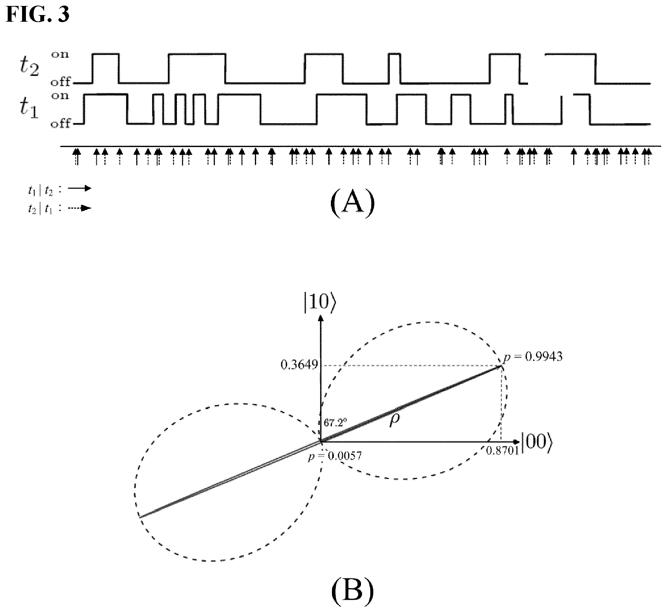

FIG. 3A is an example showing a transcriptional state of two genes.

In FIG. 3A, $t_1$ and $t_2$ represent the transcriptional state of each gene in the form of a plot. The two genes have four orthogonal (mutually exclusive) basis transcriptional states ($|00\rangle$, $|01\rangle$, $|10\rangle$, $|11\rangle$).

At any time point, the transcriptional state of one gene and the transcriptional state of another gene may be expressed as a pre-given conditional state ($t_1|t_2$ or $t_2|t_1$). In FIG. 3A, the arrows indicate the time points when the basis transcriptional state is measured. The color of the arrow indicates observation time points of each conditional state. For example, at a first observation time point of $t_1|t_2$, the basis transcriptional state is $|00\rangle$ with $t_1$ being "off" and $t_2$ also being "off." At a second observation time of $t_1|t_2$, the basis transcriptional state is $|11\rangle$. The basis transcriptional state of each observation point of $t_2|t_1$ can be determined in the same way. The results calculated by counting each basis transcriptional state for FIG. 3A are shown in Table 1 below.

[Table 1]

|  | $|00\rangle$ | $|01\rangle$ | $|10\rangle$ | $|11\rangle$ |
|---|---|---|---|---|
| $t_1 \mid t_2$ | 15 | 5 | 8 | 4 |
| $t_2 \mid t_1$ | 20 | 5 | 7 | 5 |

When Table 1 is formatted as a matrix, the transcriptional state of the entire genome becomes $T^T$. Therefore, the density matrix ρ has a trace of $TT^T$ normalized to 1 and is as follows.

[Equation 2]

$$\rho = \begin{matrix} & \langle 00| & \langle 01| & \langle 10| & \langle 11| \\ |00\rangle & \begin{bmatrix} 0.7539 & 0.2111 & 0.3136 & 0.1930 \\ |01\rangle \\ |10\rangle \\ |11\rangle \end{bmatrix} & \end{matrix}$$

$$\rho = \begin{matrix} & \langle 00| & \langle 01| & \langle 10| & \langle 11| \\ |00\rangle \\ |01\rangle \\ |10\rangle \\ |11\rangle \end{matrix} \begin{bmatrix} 0.7539 & 0.2111 & 0.3136 & 0.1930 \\ 0.2111 & 0.0603 & 0.0905 & 0.0543 \\ 0.3136 & 0.0905 & 0.1363 & 0.0808 \\ 0.1930 & 0.0543 & 0.0808 & 0.0495 \end{bmatrix}$$

Unlike $\rho^2$, the density matrix ρ is not a pure state. ρ is an average of the density matrix $\rho_1$ of $t_1|t_2$ and the density matrix $\rho_2$ of $t_2|t_1$, which is a mixed state. The dwelling probability of the basis transcriptional state $|00\rangle$ is the largest, and the dwelling probability for the remaining basis states is small.

A first eigenvector of ρ is $0.8701 |00\rangle+0.2450 |01\rangle+0.3649 |10\rangle+0.2230 |11\rangle$, and an eigenvalue is 0.9943. FIG. 3B is the result of projecting a density matrix onto a plane composed of basis transcriptional states with high dwelling probability in FIG. 3A. FIG. 3B is the result of projecting the density matrix ρ onto a plane composed of $|00\rangle$ which has the largest dwelling probability, and $|10\rangle$ which has the second largest dwelling probability.

In this example, the von Neumann entropy of $Tr(\rho\log\rho)$ of the calculated ρ is 0.05 bits. Meanwhile, the dwelling probability p that any transcriptional state t contributes to the density matrix ρ is defined as Equation 3 below.

[Equation 3]

$$p = \langle t|\rho|t\rangle$$

In the example of FIG. 3, the dwelling probabilities that the transcriptional states $t_1$ and $t_2$ of two genes contribute to the activity of the genome are calculated according to Equation 3 and are 0.9859 and 0.9906.

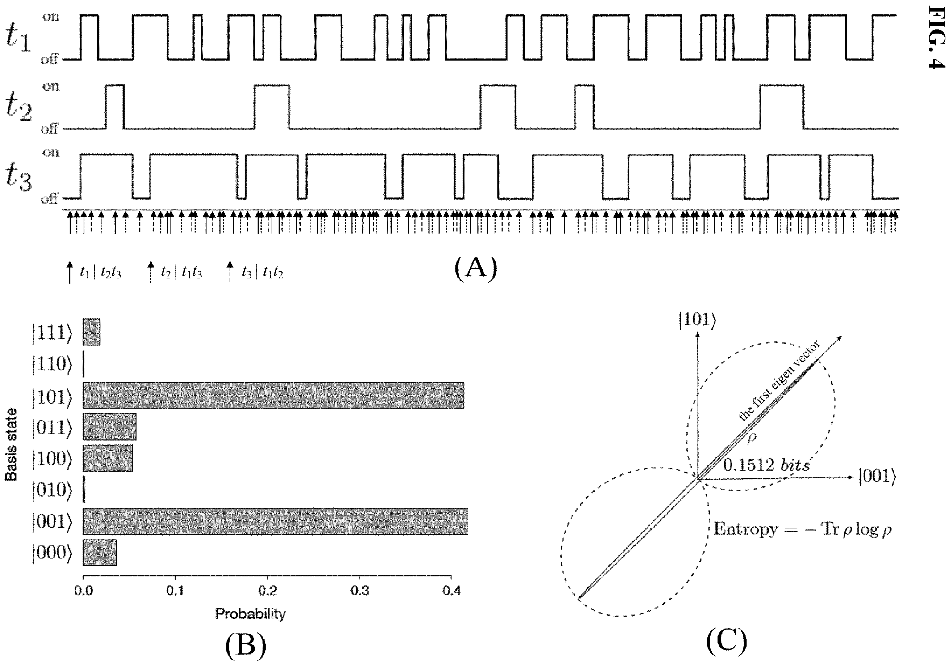

Furthermore, when the activity of genes in a genome composed of three genes is analyzed using the same method, it is as follows. FIG. 4A is an example showing a transcriptional state of three genes. FIG. 4A is an example showing $t_1$, $t_2$, and $t_3$ which are the transcriptional states of three genes. In FIG. 3A, the arrows indicate the time points when the basis transcriptional state is measured. The results of counting the conditional states $t_1|t_2t_3$, $t_2|t_1t_3$, and $t_3|t_1t_2$ for three genes are shown in Table 2 below.

[Table 2]

|  | $t_1|t_2t_3$ | $t_2|t_1t_3$ | $t_3|t_1t_2$ |
|---|---|---|---|
| $|000\rangle$ | 4 | 4 | 3 |
| $|001\rangle$ | 15 | 13 | 9 |
| $|010\rangle$ | 4 | 1 | 1 |
| $|100\rangle$ | 4 | 6 | 3 |
| $|011\rangle$ | 5 | 6 | 2 |
| $|101\rangle$ | 17 | 12 | 6 |
| $|110\rangle$ | 0 | 0 | 1 |
| $|111\rangle$ | 4 | 1 | 2 |

The transcriptional state vectors of three genes are shown in Equation 4 below.

[Equation 4]

$$|t_1\rangle = 4|000\rangle + 15|001\rangle + 4|100\rangle + 5|011\rangle + 17|101\rangle + 4|111\rangle$$

$$|t_2\rangle = 4|000\rangle + 13|001\rangle + |010\rangle + 6|100\rangle + 6|011\rangle + 12|101\rangle + |111\rangle$$

$$|t_3\rangle = 3|000\rangle + 9|001\rangle + |010\rangle + 3|100\rangle + 2|011\rangle + 6|101\rangle + |110\rangle + 2|111\rangle$$

The density matrices $\rho_1$, $\rho_2$, and $\rho_3$ of each gene are calculated as $|t_1\rangle\langle t_1|$, $|t_2\rangle\langle t_2|$ and $|t_3\rangle\langle t_3|$, respectively. The density matrix $\rho$ of the entire genome may be calculated as the arithmetic mean of the density matrices of individual genes. The density matrix $\rho$ of the entire genome is given in Equation 5 below.

[Equation 5]

$$\rho = \begin{array}{c} \\ \langle000| \quad \langle001| \quad \langle010| \quad \langle100| \quad \langle011| \quad \langle101| \quad \langle110| \quad \langle111| \\ \begin{array}{c} |000\rangle \\ |001\rangle \\ |010\rangle \\ |100\rangle \\ |011\rangle \\ |101\rangle \\ |110\rangle \\ |111\rangle \end{array} \begin{bmatrix} 0.0361 & 0.1225 & 0.0062 & 0.0432 & 0.0441 & 0.1181 & 0.0026 & 0.0229 \\ 0.1225 & 0.4185 & 0.0194 & 0.1454 & 0.1507 & 0.4097 & 0.0079 & 0.0802 \\ 0.0062 & 0.0194 & 0.0018 & 0.0079 & 0.0070 & 0.0159 & 0.0009 & 0.0026 \\ 0.0432 & 0.1454 & 0.0079 & 0.0537 & 0.0546 & 0.1392 & 0.0026 & 0.0247 \\ 0.0441 & 0.1507 & 0.0070 & 0.0546 & 0.0573 & 0.1489 & 0.0018 & 0.0264 \\ 0.1181 & 0.4097 & 0.0159 & 0.1392 & 0.1489 & 0.4132 & 0.0053 & 0.0811 \\ 0.0026 & 0.0079 & 0.0009 & 0.0026 & 0.0018 & 0.0053 & 0.0009 & 0.0018 \\ 0.0229 & 0.0802 & 0.0026 & 0.0247 & 0.0264 & 0.0811 & 0.0018 & 0.0185 \end{bmatrix} \end{array}$$

FIG. 4B is an example of a histogram for the dwelling probability of the basis transcriptional state in FIG. 4A. The dwelling probabilities of each basis transcriptional state are listed along the diagonal of the density matrix $\rho$. $|001\rangle$ and $|101\rangle$ are similar. In addition, the dwelling probability of the basis transcriptional state is non-uniform, suggesting that the degree to which three genes were transcribed is correlated.

The eigenvalues of the density matrix $\rho$ are $9.8130\times10^{-1}$, $1.2800\times10^{-2}$, and $6.0000\times10^{-3}$, with the rest close to 0. Even if the dwelling probability of the basis transcriptional state is uniform, it cannot be said that the transcriptional state is uniform. The transcriptional state may be uniform only when it has a uniform probability distribution in all directions of the space formed by the basis transcriptional state. Therefore, the uniformity of the transcriptional state may be confirmed by the uniformity of the eigenvalues.

FIG. 4C is a diagram showing the results of projecting the density matrix onto the plane composed of the basis transcriptional states with high dwelling probability in FIG. 3A. FIG. 4C is the result of projecting the density matrix $\rho$ onto the plane composed of two basis transcriptional states $|001\rangle$ and $|101\rangle$ with high dwelling probability. As described above, the non-uniformity of the eigenvalues is very large and the von Neumann entropy is also small at 0.1512 bits.

Hereinafter, the above-described process is generalized to describe the transcriptional activity of a genome composed of n genes. A basis state vector of a genome with n genes may be denoted by $|j_1 j_2 \cdots j_n\rangle$. Here $j_k \in \{0,1\}$ and k=1,...,n. The genome with n genes has a total of $2^n$ basic state vectors. Each of the n genes has a basis state vector with orthonormal characteristics. For convenience of description, the basic state vector is denoted by $|\psi_1\rangle,...,|\psi_2{}^n\rangle$. The transcriptional state T of the genome composed of n genes is expressed in Equation 6 below.

[Equation 6]

$$
T^{\mathsf{T}} = 
\begin{array}{c}
t_1|t_2\cdots t_n \\
t_2|t_1\cdots t_n \\
\vdots \\
t_n|t_1\cdots t_{n-1}
\end{array}
\begin{array}{cccccc}
|\psi_1\rangle & |\psi_2\rangle & |\psi_3\rangle & \cdots & |\psi_{2^n}\rangle \\
\left[ \begin{array}{ccccc}
a_{11} & a_{12} & a_{13} & \cdots & a_{12^n} \\
a_{21} & a_{22} & a_{23} & \cdots & a_{22^n} \\
\vdots & \vdots & \vdots & \ddots & \vdots \\
a_{n1} & a_{n2} & a_{n3} & \cdots & a_{n2^n}
\end{array} \right]
\end{array}
$$

Therefore, the density matrix $\rho$ is $TT^{\mathsf{T}}$, and the dimensions of the density matrix are $2^n \times 2^n$. The von Neumann entropy of the density matrix $\rho$ is $-\text{Tr}(p\log\rho)$, which means the non-uniformity of the probability distribution. This entropy may be an average information amount generated by the genome. The eigenvector $|v1\rangle$ with the largest eigenvalue of $\rho$ defines the attributes of the information generated by the genome.

The genetic program contained in the genome generates information through the transcriptional state T. An accurate mathematical analysis of the process by which the generated information is transmitted to mRNAis important. In genomes composed of n genes in total, a genome space where the transcriptional state is described is a space of

$$\mathbb{R}^{2^n},$$

while the mRNA corresponding to a channel through which information is transmitted is a gene space of $\mathbb{R}^n$. The genome space refers to a Hilbert space having the basis state vector of the genome as a coordinate axis.

A real space is a three-dimensional space in the real world where chemical reactions, such as production of specific proteins through the activity of genes, occur in living organisms. Therefore, it is impossible to directly access the genome space to determine the activity of the genome. Therefore, a method of converting a genome space into a sample space of gene expression data and analyzing the sample space is needed. The sample space is an m-dimensional space where each sample is defined as a unit vector.

The spatial conversion process uses two matrices U and $\Sigma$ to reduce the dimensionality while minimizing the loss of transmitted information.

$\Sigma$ is a matrix for converting the genome space into the gene space and is shown in Equation 7 below. $\Sigma$ is composed of $2^n$ rows and n columns with the diagonal elements up to the top n rows being 1 and the remaining elements being 0.

[Equation 7]

$$
\Sigma = 
\begin{bmatrix}
1 & 0 & \cdots & 0 \\
0 & 1 & \cdots & 0 \\
\vdots & \vdots & \ddots & \vdots \\
0 & 0 & \cdots & 1 \\
0 & 0 & \cdots & 0 \\
\vdots & \vdots & \ddots & \vdots \\
0 & 0 & \cdots & 0
\end{bmatrix}
$$

Meanwhile, the matrix $\overset{2^n \times 2^n}{U}$ is a rotation matrix and rotates $\overset{2^n \times n}{T}$ as shown in Equation 8 below.

[Equation 8]

$$T'^{\top} = T^{\top} U$$

The density matrix $\overset{2^n \times 2^n}{\rho}$ of the transcriptional state T has $2^n$ eigenvectors ($|v_1\rangle,...,|v_{2n}\rangle$) and eigenvalues ($\lambda_1,...,\lambda_{2n}$). Here, $\lambda_1 > \cdots > \lambda_{2n}$. Therefore, when U is composed of the eigenvectors of $\rho$, a basis vector of T' becomes equal to the eigenvectors of $\rho$. The matrix $\Sigma$ projects T' of

$$\mathbb{R}^{2^n}$$

onto $\mathbb{R}^n$. Therefore, the eigenvalues ($\lambda'_1, \ldots, \lambda'_n$) of the density matrix $\overset{n \times n}{\rho'}$ of the projected $T'^{\top}\Sigma$ approach ($\lambda_1,...\lambda_n$) because $\lambda_{n+1} \approx 0$ when the entropy of $\rho$ is sufficiently small. Therefore, the entropy of $\rho'$ approaches $-\sum_{\alpha=1}^{n} \lambda_\alpha \log \lambda_\alpha$ and also approaches the entropy of T. Therefore, information generated by eukaryotic genomes may be transmitted to mRNAs with minimal loss.

In general, the basis for obtaining measured values of the transcriptional state from the gene expression data sets of samples generated by genome analysis techniques such as next-generation sequencing (NGS) is that the same scenario is activated in the genomes of the same type of sample group. When the genome loses functions, the transcription will become dependent on transcriptional stimulation from the proteome. Therefore, the variation between samples is bound to increase. It is important to compare the entropy calculated from the gene expression data set in the same type of sample group with the entropy of the transcription state.

The gene expression data set may be interpreted as the distribution of n genes in the R^m sample space and is dependent on the transcriptional state T of the genome. As described above, T was converted into $T^{\top}\Sigma$ in the gene space of $\mathbb{R}^n$. In this process, the sample selection means the conversion from the gene space to the sample space, and

$$\overset{n \times m}{\Sigma}(ge/s)$$

is mediated between the two spaces. Therefore, $\Sigma\Sigma^{(ge/s)}$ is mediated between the genome space and the sample space. This matrix is composed of $2^n$ rows and m columns, with only the diagonal elements of the first m rows being 1 and the remaining elements being 0.

Therefore, the eigenvalues of the density matrix in the sample space approach ($\lambda_1,...,\lambda_m$), when the entropy of the transcriptional state is sufficiently small. Therefore, the entropy calculated in the sample group approaches $-\sum_{\alpha=1}^{m} \lambda_\alpha \log \lambda_\alpha$. However, when $\lambda_{m+1} \approx 0$, the entropy of the gene expression data set approaches the entropy of the transcriptional state.

The genome of eukaryotic cells including human cells has scenarios of cell operation and activates necessary scenarios depending on the type of tissue. Therefore, the activated scenarios in the same tissue may be determined by the entropy of the transcriptional state. The contents provide a mathematical basis for calculating the entropy of the transcriptional state from the gene expression data set of the sample group.

Generally, multiple genes are involved in the expression of one trait in eukaryotic cells. When a group of genes involved in the expression of a specific trait is organized within the genome and regulates the expression of the trait, the mRNAs produced by the gene group should encode information for expression and regulation.

In FIG. 1, the analysis device analyzed the sample by analyzing the gene set extracted according to the GO term for a specific trait. The gene set extracted by the GO term is called a target gene set. The analysis device analyzes samples by comparing reference data and sample data for a set of target genes. In this case, the characteristics of the target gene set are calculated based on the entropy, SP, LOR, etc., according to the above-described genomic state.

Hereinafter, the researchers describe experiments conducted using public data based on the GO term, the entropy of the transcriptional state, the SP, the LOR, etc. The data processing process or calculations described below was performed using an analysis device (computer device).

The researchers selected GO terms related to indefinite cell division which is one of the characteristic traits of tumors. By selecting GO terms, related information may be acquired by querying a database (DB) that provides GO information. The researchers extracted genes involved in the life metabolism of genes from each selected GO term. As a result, the researchers may define associated gene groups including one or more genes associated with each GO term. The genes included in each of these gene groups are the results of individual research in the corresponding field, and each gene may not include all genes necessary for the expression of a trait, but the degree of involvement may vary depending on the type of tissue. Therefore, the researchers set the minimum number of genes for which the information amount may be calculated relatively stably to 10, and selected a GO term that includes genes greater than the set number. The standard for the number of genes for selecting the GO terms may also be determined experimentally. Table 3 below shows the GO terms selected by the researchers in association with the infinite cell division which is one of the characteristic traits of tumors.

[Table 3]

| No. | GO term | Number of genes |
|---|---|---|
| 1 | anaphase-promoting complex-dependent proteasomal ubiquitin-dependent protein catabolic process | 80 |
| 2 | cell cycle | 1038 |
| 3 | cell cycle arrest | 224 |
| 4 | cell cycle checkpoint | 39 |
| 5 | cell cycle phase transition | 12 |
| 6 | cell division | 163 |
| 7 | cellular response to DNA damage stimulus | 160 |
| 8 | centriole replication | 17 |
| 9 | centromere-specific nucleosome assembly | 21 |
| 10 | centrosome organization | 23 |
| 11 | chromatin modification | 97 |
| 12 | chromatin organization | 103 |
| 13 | chromatin remodeling | 79 |
| 14 | chromosome condensation | 30 |
| 15 | chromosome organization | 19 |
| 16 | chromosome segregation | 73 |
| 17 | DNA damage checkpoint | 83 |
| 18 | DNA damage response, signal transduction by p53 class mediator | 114 |
| 19 | DNA damage response, signal transduction by p53 class mediator resulting in transcription of p21 class mediator | 17 |
| 20 | DNA duplex unwinding | 43 |
| 21 | DNA repair | 293 |
| 22 | DNA replication | 269 |
| 23 | DNA replication checkpoint | 11 |
| 24 | DNA replication initiation | 29 |
| 25 | DNA replication-independent nucleosome assembly | 11 |
| 26 | DNA strand elongation involved in DNA replication | 31 |

(continued)

| No. | GO term | Number of genes |
|---|---|---|
| 27 | DNA unwinding involved in DNA replication | 11 |
| 28 | DNA-dependent DNA replication | 37 |
| 29 | double-strand break repair | 126 |
| 30 | double-strand break repair via homologous recombination | 67 |
| 31 | establishment of mitotic spindle orientation | 13 |
| 32 | exit from mitosis | 19 |
| 33 | G1/S transition of mitotic cell cycle | 210 |
| 34 | G2 DNA damage checkpoint | 32 |
| 35 | G2/M transition of mitotic cell cycle | 164 |
| 36 | kinetochore assembly | 11 |
| 37 | metaphase plate congression | 15 |
| 38 | mismatch repair | 27 |
| 39 | mitotic cell cycle | 617 |
| 40 | mitotic cell cycle checkpoint | 27 |
| 41 | mitotic chromosome condensation | 13 |
| 42 | mitotic cytokinesis | 18 |
| 43 | mitotic G2 DNA damage checkpoint | 11 |
| 44 | mitotic metaphase | 20 |
| 45 | mitotic metaphase plate congression | 11 |
| 46 | mitotic nuclear division | 230 |
| 47 | mitotic nuclear envelope disassembly | 37 |
| 48 | mitotic sister chromatid cohesion | 11 |
| 49 | mitotic sister chromatid segregation | 21 |
| 50 | mitotic spindle assembly checkpoint | 32 |
| 51 | mitotic spindle organization | 23 |
| 52 | negative regulation of cell cycle | 55 |
| 53 | negative regulation of DNA replication | 22 |
| 54 | negative regulation of G1/S transition of mitotic cell cycle | 22 |
| 55 | negative regulation of ubiquitin-protein ligase activity involved in mitotic cell cycle | 66 |
| 56 | nucleotide-excision repair | 79 |
| 57 | nucleotide-excision repair, DNA gap filling | 20 |
| 58 | positive regulation of cell cycle | 50 |
| 59 | positive regulation of DNA repair | 24 |
| 60 | positive regulation of DNA replication | 38 |
| 61 | positive regulation of G2/M transition of mitotic cell cycle | 14 |
| 62 | positive regulation of mitosis | 29 |
| 63 | positive regulation of mitotic cell cycle | 31 |
| 64 | regulation of cell cycle | 193 |

(continued)

| No. | GO term | Number of genes |
|---|---|---|
| 65 | regulation of cell cycle arrest | 36 |
| 66 | regulation of DNA recombination | 14 |
| 67 | regulation of DNA replication | 70 |
| 68 | regulation of double-strand break repair via homologous recombination | 13 |
| 69 | regulation of exit from mitosis | 13 |
| 70 | regulation of G1/S transition of mitotic cell cycle | 45 |
| 71 | regulation of G2/M transition of mitotic cell cycle | 25 |
| 72 | regulation of mitosis | 55 |
| 73 | sister chromatid segregation | 24 |
| 74 | spindle assembly | 77 |
| 75 | spindle assembly checkpoint | 40 |
| 76 | spindle assembly involved in mitosis | 11 |
| 77 | spindle checkpoint | 12 |
| 78 | spindle organization | 42 |

For the selected 78 GO term gene groups, the von Neumann entropy was calculated to measure the activity in each tissue. The entropy calculation for the gene set is as described above. Describing briefly, the researchers calculated a density matrix $\rho_i = G_i^{\top} G_i / \mathrm{Tr}\left(G_i^{\top} G_i\right)$ of gene expression data Gi of a gene group $g_i$ of an $i^{th}$ GO term. The researchers calculated the entropy for $g_i$ by calculating $-\rho_i \log 2 \rho_i$. In addition, the same number of genes included in the gene group of one GO term was randomly selected and the entropy was calculated in the same way. The process was performed 1,000 times and the average was calculated to calculate the random entropy for all 78 GO terms.

The researchers used publicly available gene expression data sets. The gene expression data set used data from six types of cancer tissues such as breast cancer (BRCA), colon cancer (COAD), rectal cancer (READ), lung adenocarcinoma (LUAD), lung squamous cell carcinoma (LUSC), and ovarian cancer (OV), and two types of normal tissues such as normal breast tissue (BRNO) and normal colon tissue (CONO). BRCA, etc., refers to a data set in which a gene expression level from the corresponding tissue was measured by The Cancer Genome Atlas (TCGA) and released to the public for academic research.

The researchers performed a one-way ANOVA test to verify statistical significance between the entropy and random entropy of 78 GO term gene groups in samples of each tissue.

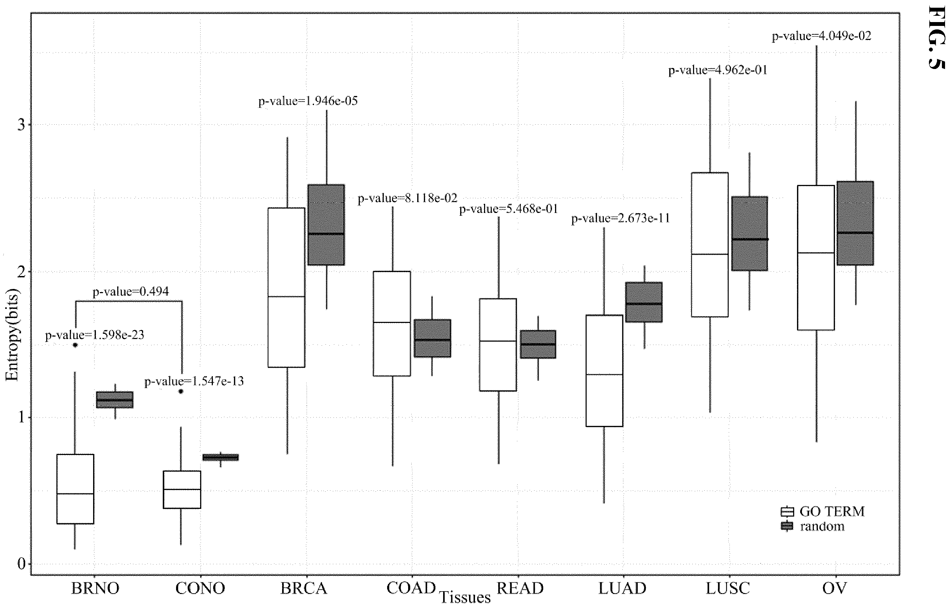

The gene set (hereinafter referred to as GO term genetic data) selected as the GO terms includes genes selected as the entire GO terms. FIG. 5 is a diagram showing the results of a one-way ANOVA test on the entropy of a cell cycle-related gene ontology (GO) term gene set.

FIG. 5 shows the results of comparing the entropy of the GO term gene set with the randomly selected gene set among the genes of each tissue sample. In FIG. 5, the "GO term" represents the entropy (hereinafter referred to as the GO term entropy) of the GO term gene set, and the "random" represents the random entropy of the gene set randomly selected from the corresponding tissue sample. The randomly selected gene set is composed of the same number of genes as the number of genes in the GO term gene set of the corresponding tissue sample.

Looking at FIG. 5, the GO term entropy distribution related to the cell cycle in normal breast tissue (BRNO) and normal colon tissue (CONO) was significantly lower than the entropy distribution of the GO term gene sets in tumor tissues (p-value<$10^{-19}$). On the other hand, there was no significant difference in the entropy between the two normal tissues BRNO and CONO (p-value=0.494). Meanwhile, there was a significant difference between the GO term entropy and the random entropy in normal tissues. However, there was no significant difference in the GO term entropy distribution and the random entropy in tumor tissue, or even if there was the difference, the difference was not as large as in normal tissue. These results indicate that the cell cycle-related genomic system is disrupted in most tumor tissues. However, in LUAD, the p-value between the GO term entropy distribution and the random entropy distribution was $2.673 \times 10^{-11}$, which shows that the cell cycle-related genomic system functions relatively normally.

These results suggest that the function of the cell cycle-related genomic system, which operates very well in normal tissues, varies depending on the malignancy of the tumor. In some tumors, this means that the cell cycle-related genomic system has suffered functional destruction at the level of a random gene set.

Meanwhile, to confirm whether there were differences between tissues in the activity of the cell cycle-related GO term gene set, the entropy of 78 GO terms was calculated in 8 types of tissues. First, linear regression was performed between the GO term entropies of the two normal tissues BRNO and CONO. As a result, a slope was 0.506, a y-intercept was 0.241, and a coefficient of determination $r^2$ was 0.558. In addition, the GO term entropy in both tissues had mostly low values (BRNO: 0.088 to -1.147 bits, CONO: 0.101 to 0.767 bits). Therefore, it can be seen that the activity of the cell cycle-related GO term gene set in the two normal tissues is in a normal state, but the operation of cell cycle regulation is different.

Table 4 below shows the results of performing linear regression on the GO term entropies of each tumor tissue with respect to the cell cycle-related GO term entropy of BRNO which is normal tissue. As the result of performing linear regression on BRCA and LUAD with respect to BRNO, unlike other tumor tissues, the slope was greater than 1 and the y-intercept was smaller than 1. In addition, the coefficients of determination $r^2$ were the first and second largest. These results suggest that the cell cycle-related genomic systems of these two tissues were relatively less disrupted. On the other hand, the low $r^2$ and slope and the large y-intercept in the remaining four tumor tissues COAD, READ, LUSC, and OV showed that the cell cycle-related genomic system is severely disrupted.

[Table 4]

| Tissue | Range of Entropy (bit) | | $r^2$ | Slope | Intercept | $p$-value |
|---|---|---|---|---|---|---|
| | $x$ | $y$ | | | | |
| BRCA | 0.093 - 1.368 | 0.916 - 2.464 | 0.721 | 1.270 | 0.805 | $9.6 \times 10^{-23}$ |
| COAD | 0.089 - 1.311 | 0.617 - 2.172 | 0.292 | 0.613 | 1.058 | $3.4 \times 10^{-7}$ |
| READ | 0.093 - 1.327 | 0.714 - 2.559 | 0.320 | 0.645 | 1.011 | $6.9 \times 10^{-8}$ |
| LUAD | 0.089 - 1.274 | 0.991 - 2.483 | 0.640 | 1.142 | 0.674 | $1.6 \times 10^{-18}$ |
| LUSC | 0.092 - 1.332 | 0.966 - 2.543 | 0.312 | 0.718 | 1.445 | $1.1 \times 10^{-7}$ |
| OV | 0.096 - 1.356 | 1.060 - 2.508 | 0.320 | 0.746 | 1.348 | $6.7 \times 10^{-8}$ |

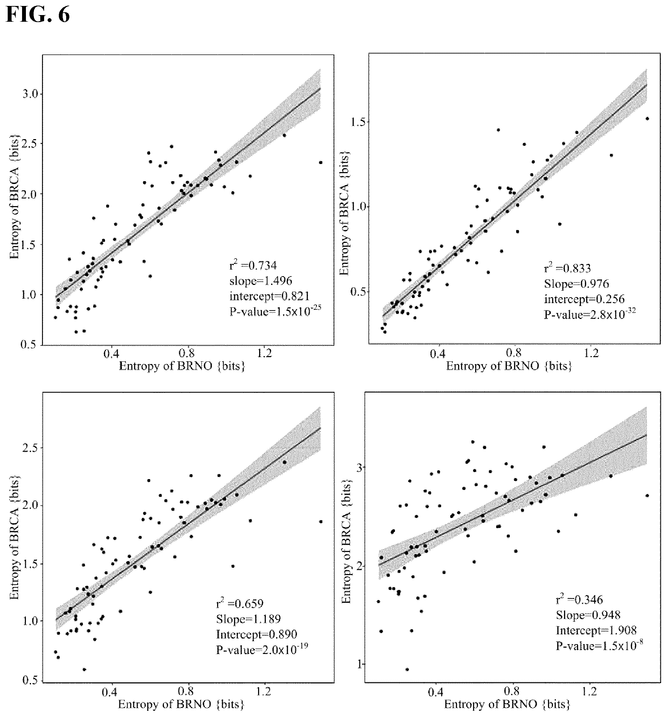

To more precisely investigate the variation of the GO term entropy of tumor tissues with respect to normal tissues, the tumor tissue samples were divided according to the size of the SP to calculate the GO term entropy. FIG. 6 is an example of separately analyzing the correlations between the GO term entropy of BRCA and the GO term entropy of BRNO according to the sample probability of BRCA. FIG. 6 shows the results of calculating the cell cycle-related GO term entropy by dividing the breast cancer samples according to the size of the SP and performing linear regression on the entropy of the normal breast tissue. First, 28 samples were randomly selected from among a total of 248 BRCA samples to calculate the cell cycle-related GO term entropy and perform linear regression on the entropy of BRNO. As a result, the coefficient of determination $r^2$ was relatively large (0.734), but the slope (1.496) and the y-intercept (0.821) increased. In three sample groups (high SP, mid SP, low SP) into the breast cancer samples were divided based on the SP, the coefficient of determination $r^2$ tended to decrease rapidly to 0.833, 0.659, and 0.346, and all the slopes had values close to 1. However, in the low SP sample group, the coefficient of determination is small, so linearity has little meaning. On the other hand, the y-intercept increased to 0.256, 0.890, and 1.908. In other words, as the SP decreases, the cell cycle-related GO term entropy increases, suggesting the loss of function. These results are consistent with Table 3-1 in which the coefficients of determination and slopes of the COAD, READ, LUSC, and OV entropies decrease and the y-intercept increases.

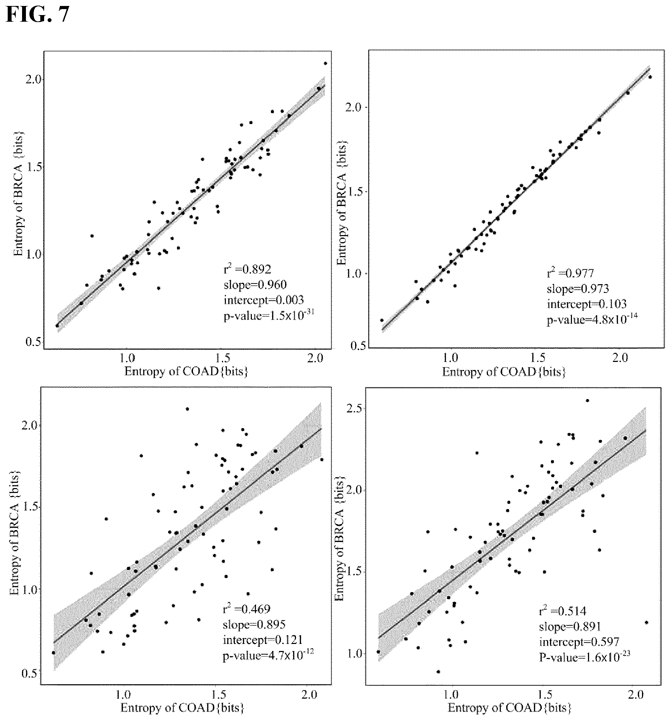

Meanwhile, in Table 4, the slopes and y-intercepts of the COAD and READ entropies with respect to the entropy of BRNO have approximate values. FIG. 7 is an example of analyzing the correlations between the GO term entropy of COAD and the GO term entropies of READ, COAD, BRCA, and LUSC. FIG. 7 shows the results of performing linear regression on the READ, COAD, BRCA, and LUSC entropies with respect to the cell cycle-related GO term entropy of COAD. Since the size of the entropy refers only to the information amount, the attributes of direction are excluded. Nevertheless, the slope and the y-intercept are close 1 and 0, respectively. In the case of the coefficient of determination $r^2$, the coefficient of determination of READ (0.892) is slightly lower than the coefficient of determination of COAD itself (0.977), but when considering that it is significantly higher than the coefficient of determination of BRCA (0.469) or the coefficient of determination of LUSC (0.514), this suggests COAD and READ are tumors with quite similar characteristics.

Relative entropy is a measured value that may show the degree to which one system differs from another. The researchers calculated relative entropy to confirm whether the activity of the genomic system of individual GO term gene sets differed between tissues.

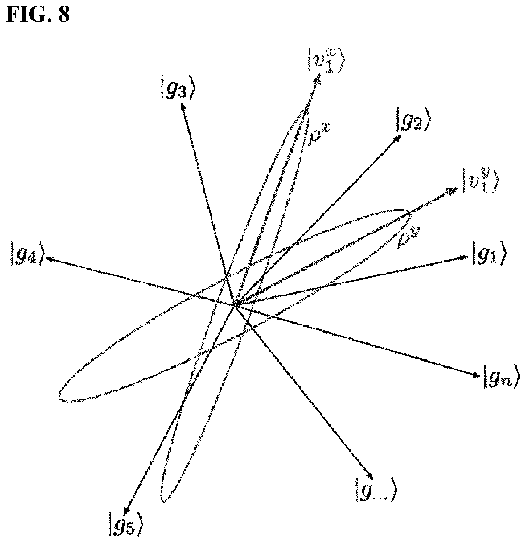

FIG. 8 is an example of the relative entropy of tissue y with respect to tissue x. For any GO term gene set composed of n genes in total, the relative entropy of one tissue x with respect to another tissue y shows the differences in attributes between two tissues. In the n-dimensional gene space, tissue x and tissue y have density matrices $\rho^x$ and $\rho^y$, respectively. First eigenvectors ' and ' of the respective density matrices are correlated with the attribute by which the gene set of the corresponding GO term i is active in each tissue. In other words, the relative entropy S ($\rho^y \parallel \rho^x$) expressed in Equation 9 below represents the degree to which the activity in tissue y varies relative to the gene set of the corresponding GO term i being activated in tissue x.

[Equation 9]

$$S\left(\rho^y \| \rho^x\right) = \mathrm{Tr}\left(\rho^y \left(\log \rho^y - \log \rho^x\right)\right)$$

For any GO term gene set, the factors that increase the relative entropy of another tissue with respect to one tissue are:. First, the entropies in the two tissues are different. Second, the biological attributes of the corresponding GO term gene sets in the two tissues are different. In FIG. 8, compared to the density matrix of tissue x, the density matrix of tissue y has a shape closer to a circle, which means that entropy increases. In addition, it shows that the first eigenvector of tissue y has a different direction compared to the first eigenvector of tissue x. Meanwhile, angular divergence (AD) means that the biological attributes are different in the activation of the corresponding GO term gene set.

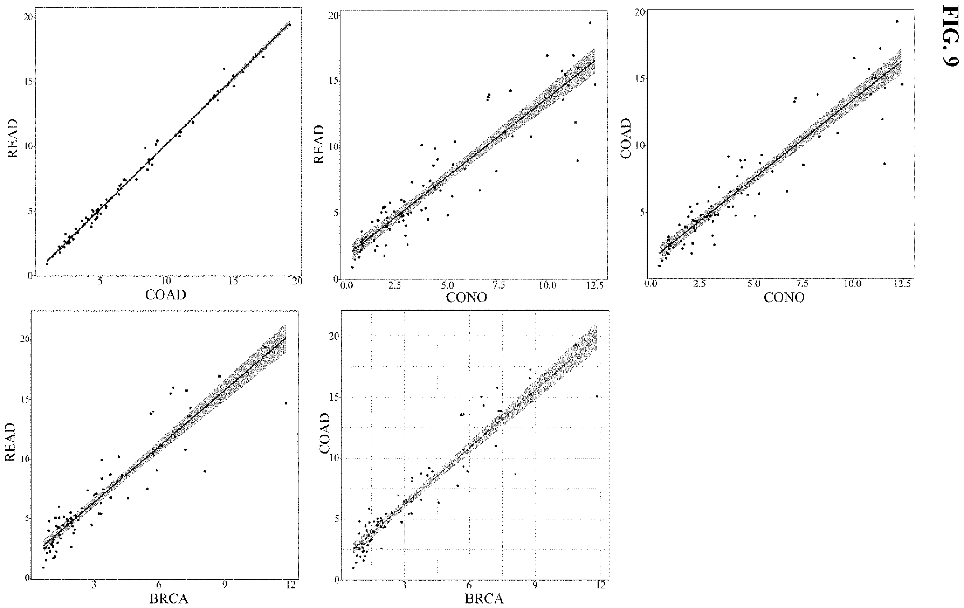

First, to secure the reliability of the relative entropy and AD, the relative entropy and AD of each of the other tissues BRCA, COAD, READ, LUAD, LUSC, OV, and CONO with respect to BRNO for the cell cycle-related GO term gene set were calculated. In addition, the results were analyzed by performing a linear regression on the calculated relative entropy of each tissue with respect to BRNO. FIG. 9 is an example of analyzing the correlations between the GO term relative entropy of each tissue with respect to BRNO. FIG. 9 shows the results of calculating the cell cycle-related GO term relative entropy of each tissue with respect to normal breast tissue and performing a linear regression between the GO term relative entropies of two randomly selected tissues with respect to BRNO. $r^2$ between the relative entropies of COAD and READ, which are considered to be almost identical tissues in biological terms, is 0.992, which is close to 1, and the slope is also 1.016, which is close to 1. In addition, the intercept is 0.128, which is close to 0. On the other hand, the $r^2$, slope, and intercept between CONO and READ were 0.802, 1.175, and 1.948, respectively, and the $r^2$, slope, and intercept between CONO and COAD were 0.823, 1.167, and 1.747, respectively. In addition, the $r^2$, slope, and intercept between BRCA and READ were 0.8641, 1.5660, and 1.6776, respectively, and the $r^2$, slope, and intercept between BRCA and COAD were 0.8841, 1.5645, and 1.4950, respectively.

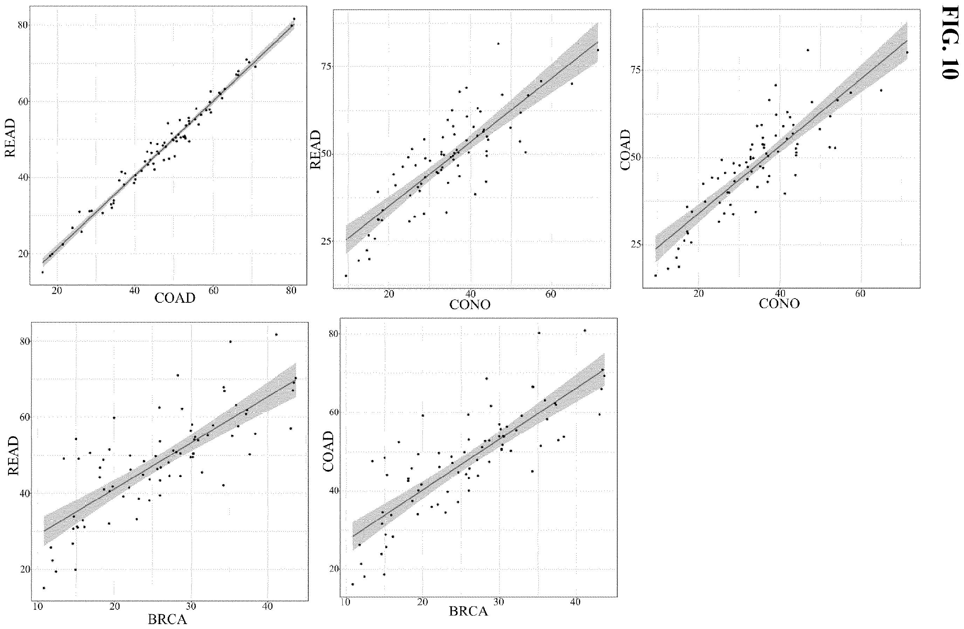

FIG. 10 is an example of analyzing the correlations between the GO term AD of each tissue with respect to BRNO. FIG. 10 shows the results of calculating the cell cycle-related GO term relative entropy of each tissue with respect to normal breast tissue and performing a linear regression between the GO term relative entropies of two randomly selected tissues with respect to BRNO. For the AD, $r^2$, slope, and intercept between COAD and READ were 0.9745, 0.9690, and 1.8874, respectively. The $r^2$, slope, and intercept between CONO and READ were 0.6762, 0.9168, and 16.8844, respectively. The $r^2$, slope, and intercept between CONO and COAD were 0.7236, 0.9662, and 14.79328, respectively. The $r^2$, slope, and intercept between BRCA and READ were 0.6023, 1.2077, and 17.0596, respectively. The $r^2$, slope, and intercept between BRCA and COAD were 0.6671, 1.2949, and 14.4069, respectively.

The above results not only prove that COAD and READ are the same type of tissue, but also means that relative entropy and AD are important indicators in defining the difference between tissues.

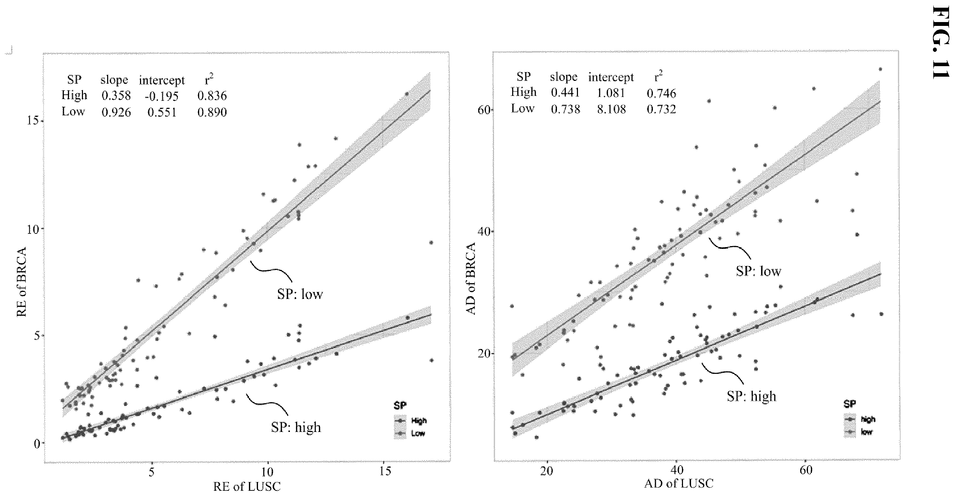

The degrees to which the genomic system of the cell cycle-related GO term loses functions in LUSC and BRCA, which have the highest degree of malignancy, were compared using relative entropy and AD. The cell cycle-related GO term relative entropy and AD with respect to BRNO in 50 samples with low SP and 50 samples with high SP among the breast cancer samples were calculated. Additionally, the cell cycle-related GO term relative entropy and AD of LUSC with respect to BRNO were calculated. FIG. 11 is an example of separately analyzing the correlations between the GO term relative entropy and AD of BRCA and the GO term relative entropy and AD of LUSC with respect to BRNO according to the SP of BRCA. FIG. 11A shows the results of performing linear regression on the cell cycle-related GO term relative entropy with respect to BRNO in the BRCA low SP sample group and high SP sample group with respect to the GO term relative entropy of LUSC. As the results of performing linear regression on the breast cancer low SP sample group and LUSC, the coefficient of determination was 0.890 and the slope was 0.926, which was close to 1. On the other hand, as the results of performing linear regression on the high SP sample group and LUSC, the coefficient of determination was 0.836 and the slope was 0.358, which was very low. FIG. 11B shows the results of performing linear regression on the cell cycle-related GO term AD with respect to BRNO in the BRCA low SP sample group and high SP sample group with respect to the GO term AD of LUSC. As the results of performing linear regression on the AD of the breast cancer low SP sample group and LUSC with respect to BRNO, the slope was 0.738, which was lower than the slope of the relative entropy. On the other hand, as the results of performing linear regression on the AD of the breast cancer high SP sample group and LUSC with respect to BRNO, the slope was 0.441, which was greater than the slope of the relative entropy. These results suggest that the relative entropy of tumor tissue with respect to normal tissue is mainly an indicator reflecting the disruption of the genomic system. In particular, the fact that the slope between the relative entropy of the breast cancer low SP sample group and the relative entropy of LUSC is close to 1 shows that breast cancer and LUSC ultimately converge into similar types of tumor cells due to the disruption of the genomic system.

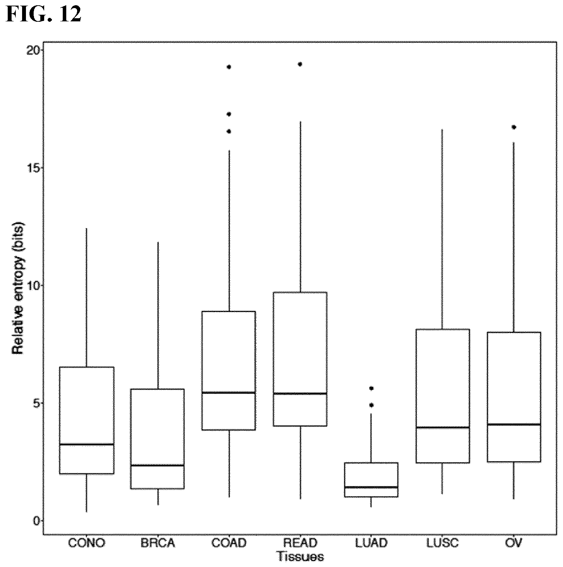

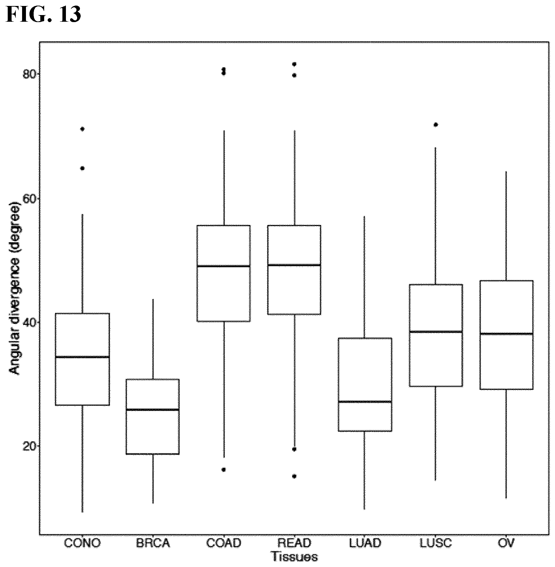

FIG. 12 is an example of comparing the GO term relative entropy of each tissue with respect to BRNO. FIG. 13 is an example of comparing the GO term AD of each tissue with respect to BRNO. Referring to FIG. 5, in most GO terms, the entropies of BRNO and CONO were almost at the same small level. However, referring to FIG. 12, the relative entropy of CONO with respect to BRNO is 0.3632 to 12.4180 bits, which is slightly greater than the relative entropy of BRCA with respect to BRNO. This means that the relative entropy increases because the AD of CONO with respect to BRNO is large.

Referring to FIG. 13, the AD of CONO with respect to BRNO is actually greater than the AD of BRCA with respect to BRNO. This suggests that the biological characteristics of the cell cycle of CONO are significantly different from those of BRNO. As described above, the cell cycle-related GO term entropy in tumors increases compared to the entropy of normal tissue. This means that the increase in both relative entropy and AD in tumors is due to both the disruption and change in attributes of cell cycle-related genomic systems. Meanwhile, referring to FIG. 5, in the case of LUAD, the distribution of the cell cycle-related GO term entropy shows a significant difference from the distribution of the random entropy (p-value=$2.673 \times 10^{-11}$), which may indicate that the genomic system of the cell cycle-related GO term is relatively stable. In FIG. 12, the relative entropy of LUAD with respect to BRNO, which is normal breast tissue, was lower than all other tissues, but in FIG. 13, the AD of LUAD with respect to BRNO was greater than that of BRCA, which is breast cancer. As a result, it can be seen that the cell cycle is relatively well regulated in LUAD, but its biological attributes are different from BRNO, and it can be assumed that the proliferation rate of cancer cells and the occurrence frequency of mutations are reduced.

GO terms have been generated as a product of various biological studies. Therefore, they have important biological and medical significance. The researchers conducted research focusing on gene expression data for breast cancer and developed a method of measuring information on deviations from a normal state of a GO term genomic system in individual patients. The corresponding indexes will be described below.

## GO Sample Probability (GSP)

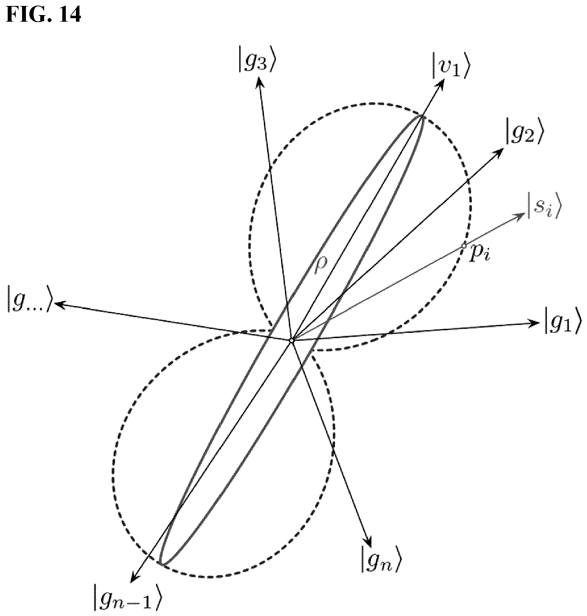

FIG. 14 is an example of displaying the probability of a sample in the gene space of a genomic system having a density matrix $\rho$. In general, in the gene space $R^n$ formed by n genes that have one GO in common, the dimensions of the density matrix $\rho$ are n×n. Therefore, $\rho$ is expressed as an ellipsoid composed of n eigenvectors and eigenvalues for each eigenvector in the gene space. In general, normal tissue samples are distributed along the eigenvector $|v_1\rangle$ with the largest eigenvalue in a given gene space. The probability $p_i$ for the density matrix $\rho$ of sample i is given by $\langle s_i|\rho|s_i\rangle/\|s_i\|^2$. $|s_i\rangle$ is the expression vector determined by the gene expression data of sample i. As $|s_i\rangle$ approaches $|v_1\rangle$, $p_i$ increases. $p_i$ is named SP. On the other hand, samples that move away from $|v_1\rangle$ deviate from a normal cluster and thus their SP decreases. That is, the degree to which the tumor sample is transformed from normal tissue may be estimated by the SP.

First, the researchers calculated the density matrix of all BRNO genes to determine the degree to which the genomic system of individual BRCA patients deviates from BRNO, and then calculated the SP in individual samples of BRNO and BRCA for the calculated density matrix.

The researchers used the gene expression data set, the clinical information, and the mutation information of 248 breast cancer (BRCA) patients released to the public for academic research by the TCGA. The clinical information includes information such as expression of an estrogen receptor (ER), a progesterone receptor (PR), and human epidermal growth factor receptor 2 (HER2), survival, and time of death for each breast cancer patient. The mutation information includes information such as a mutation generating gene, a location in the genome, and the type of mutation for each patient. In addition, the gene expression data set of 28 normal breast tissue (BRNO) samples which were pathologically determined to be normal during tissue collection from the breast cancer patients was used. The researchers cannot acquire individual patient information other than the academic and clinical information of the samples (tissues) provided by the TCGA, and therefore, all the analyzed samples (tissues) were named by serial number (e.g., BRCA patient No. 1 and the like).

The SP was the smallest at 0.155 in BRCA patient No. 4 and the largest at 0.657 in BRCA patient No. 41. The SP of patient No. 41 ranges from 0.550 to 0.791 of the SP of BRNO, which is normal breast tissue. Of course, since genes that are not activated in normal breast tissue were also included, the SP may not accurately reflect the state of the genomic system of each individual sample. However, to verify the relationship where the SP represents the malignancy information of breast cancer, as a result of verifying a difference between triple negative breast cancer (TNBC) and non-TNBC using one-way ANOVA, the SP of TNBC was significantly small (p-value=$8.61 \times 10^{-7}$).

The researchers calculated the SP of the BRNO and BRCA samples, not for all genes but for all genes in which at least one of the 78 cell cycle-related GO terms described above is included in an annotation. That is, the researchers calculated the SP in the gene space formed by the gene set sampled as the entire cell cycle-related GO term (hereinafter referred to as the entire cell cycle-related GO term gene set) for the BRNO and BRCA samples.

The maximum value of the SP for the entire cell cycle-related GO term gene set in BRCA was 0.772. This value was a value higher than the maximum value (0.657) of the SP for all the genes. However, in BRCA, the lowest value of the SP for the entire cell cycle-related GO term gene set was 0.06, which was smaller than the lowest value (0.155) of the SP for all the genes. Therefore, it suggests that the genomic system of the cell cycle is completely disrupted in the corresponding sample. In addition, the SP distribution of the TNBC sample was also skewed toward smaller values than the probability for all the genes, and showed a more significant difference (p-value=$2.98\times10^{-12}$) from the SP distribution of the non-TNBC sample. Therefore, it shows that the expression of the ER, PR, and HER2 is related to the cell cycle control function of the genomic system.

The researchers applied these principles to each cell cycle-related GO term. The researchers calculated the individual tumor sample probability for the density matrix of normal tissue in the gene space formed by a specific GO term gene set. This SP is named GSP. That is, the GSP represents the degree to which the GO term gene set deviates from normal in tumor tissue.

The researchers calculated the GSP of the BRCA samples for the density matrix of BRNO in 78 cell cycle-related GO terms. The GSP may be expressed as Equation 10 below.

[Equation 10]

$$P(s_i|G_\alpha) = \sigma_{i\alpha}^{\mathsf{T}}\rho_\alpha^{(s)}\sigma_{i\alpha},$$
$$\rho_\alpha^{(s)} = \frac{\mathbf{G}_\alpha\mathbf{G}_\alpha^{\mathsf{T}}}{tr(\mathbf{G}_\alpha\mathbf{G}_\alpha^{\mathsf{T}})},$$
$$\sigma_{i\alpha} = \frac{s_{i\alpha}}{\|s_{i\alpha}\|}$$

In Equation 10, $G_\alpha$ refers to the expression matrix of the gene set included in the specific GO term gene set $\alpha$ of normal tissue. $s_{i\alpha}$ refers to the gene expression vector included in $\alpha$ in the specific sample data $s_i$. That is, the GSP represents a genomic system variation of a specific sample tissue identified based on a specific GO term gene set of normal tissue.

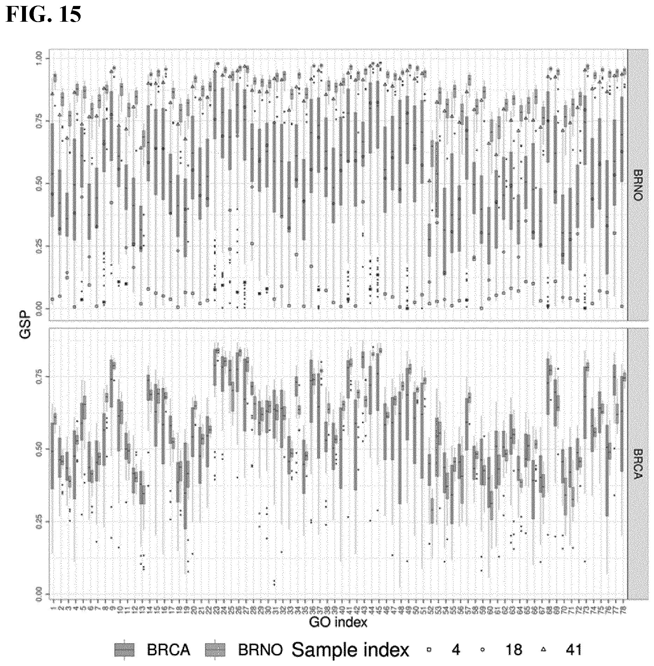

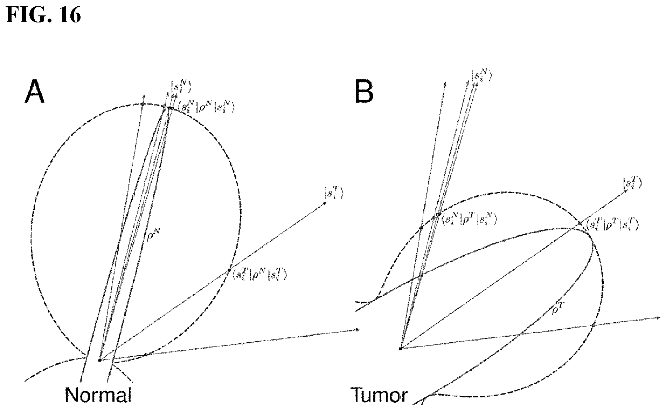

FIG. 15 is an example of calculating the GSP of the BRNO and BRCA samples for the density matrix of BRNO and the GSP of the BRNO and BRCA samples for the density matrix of BRCA in 78 cell cycle-related GO terms. In FIG. 15A, the GSP of the BRNO sample for the density matrix of BRNO has a narrow distribution range, while the GSP of the BRCA sample has a wide distribution area. To confirm this, the GSP of the BRNO sample was calculated for the density matrix of BRCA in each GO term, the same number of BRCA samples were randomly selected, and then the GSP was calculated to compare distribution areas. In FIG. 15B, the GSP distribution area of the BRNO sample for the density matrix of BRCA was reduced, but the width of the distribution was also narrower than that of the GSP of BRCA. The reason is as shown in FIG. 16.

FIG. 16 is an example of the GSP distribution of the normal tissue samples and the GSP distribution of the tumor samples for the density matrices of the normal genome and tumor genome. FIG. 16A illustrates the GSP distribution area of the normal tissue samples and the GSP distribution area of the tumor samples for the density matrix of the normal genome. FIG. 16B illustrates the GSP distribution area of the normal tissue samples and the GSP distribution area of the tumor samples for the density matrix of the tumor genome. The GSP of the normal tissue samples for the tumor genome decreases, but the GSP in both genomes is narrowly distributed. On the other hand, the GSP of the tumor sample has a wide distribution in both genomes. This wide distribution range of the GSP in the tumor sample means that various mutations in the GO term-related genomic system may occur in each tumor sample.

FIG. 16A shows that, in all GO term gene sets, the GSP distribution of the BRCA samples is significantly (p-value<$10^{-5}$) lower than the GSP distribution of the BRNO samples. In addition, the probability distribution of BRCA is wider than that of BRNO, indicating that the changed characteristics of the cell cycle differ greatly between samples. FIG. 15 displays the GSP for patients (samples) Nos. 4, 18, and 41. In the case of patient No. 41 who has a large SP among the BRCA samples, the GSP had large values in all the GO term gene sets, but showed slightly lower values than the GSP of normal breast tissue (BRNO). On the other hand, the GSP of patient No. 4 with TNBC was the lowest value or a value close to the lowest value in most GO term gene sets. In the case of patient No. 18 who is positive for the ER and PR, the GSP is greater than that of patient No. 4, but shows a lower overall value.

The researchers identified the correlation between the GSP of the cell cycle and the phenotype of breast cancer. First, the difference in the GSP distribution according to the expression of the ER, PR, and HER2 was calculated. There was a significant difference in all GO terms except for GO term No. 12 (chromatin organization) between ER+ and ER- sample groups. There was a significant difference in GSP in all 78 GO terms between the PR+ and PR- sample groups. The GO terms with the smallest p-value in both the ER and PR was No. 36 (kinetochore assembly), which were $6.01 \times 10^{-24}$ and $4.08 \times 10^{-18}$. These results mean that the genomic system of the cell cycle-related GO term is relatively preserved in tumor cells that express ERs and PRs compared to cells that do not express ERs and PRs. Therefore, ER and PR blockers may be useful in regulating the cell cycle, and even if ERs and PRs are expressed, the function of the blockers will inevitably be limited in patients with low GSP values.

The researchers also calculated the GSP for each cell cycle-related GO term between HER2+ and HER2-. Table 5 below summarizes the results of major GO terms for the HER2+ and HER2- samples.

[Table 5]

| GO index | p-value | GO |
| --- | --- | --- |
| 53 | 0.001 | negative regulation of DNA replication |
| 55 | 0.008 | negative regulation of ubiquitin-protein ligase activity involved in mitotic cell cycle |
| 67 | 0.011 | regulation of DNA replication |
| 12 | 0.019 | chromatin oranization |
| 62 | 0.033 | positive regulation of mitosis |
| 60 | 0.036 | positive regulation of DNA replication |
| 72 | 0.048 | regulation of mitosis |
| 11 | 0.049 | chromatin modification |
| 3 | 0.050 | cell cycle arrest |

Looking at Table 5, 9 GO terms showed significant differences in the GSP distribution depending on whether HER2 was expressed or not.

The expression of receptors for estrogen and progesterone, which are steroid hormones, causes fundamental changes in cell function. Therefore, the effects of ER and PR expression on HER2 expression are bound to be different. The researchers searched for GO terms with significant differences in the GSP distribution between HER2+ and HER2- when ER+ and ER-, respectively. There were 41 GO terms with significant differences in the GSP distribution depending on whether HER2 was expressed in ER+ breast cancer cells, and the GSP distribution of HER2- had a larger value than the GSP of HER2+. However, in ER-, the GSP of HER+ was distributed significantly higher than the GSP of HER2- in 39 GO terms. That is, the changes in the distribution of the GSP due to HER2 between ER+ and ER- were in opposite directions. In addition, as a result of confirming whether there was a difference in the related GO terms, although the direction of change in the distribution was opposite, only 19 GO terms (4, 14, 16, 17, 19, 22, 36, 39, 42, 46, 48, 50, 64, 66, 68, 71, 74, 75, and 77) among 41 (ER+) and 39 (ER-) were included in both. These results indicate that the ER+ and ER-breast cancer cells have completely different cell cycle biological attributes.

Various recent research techniques have made large-scale, precise tracking of mutations in genes possible. The researchers attempted to identify the correlation between the disruption of the genomic system and the occurrence of mutations in several GO terms related to the cell cycle in breast cancer. First, looking at the occurrence distribution of mutations, there were samples with unusually high frequencies. Therefore, the researchers used only samples with a mutation frequency of 312 or less to exclude outliers. The samples were classified based on the number of mutations occurring in BRCA (90) and as a result of comparing the SP distribution of the BRCA samples with respect to BRNO of the entire gene set, significantly (p-value=$1.10 \times 10^{-9}$) more mutations occurred when the SP was small. These results suggest that the degree of disruption of the genomic system of breast cancer is related to the frequency of mutations. In addition, in order to verify the relationship between the cell cycle and the occurrence of mutations, the difference in the SP distribution calculated in the same manner using only 1,944 genes included in the cell cycle-related GO term gene set was also significant (p-value=$1.58 \times 10^{-9}$), with more mutations occurring in cases where the SP was small. These results suggest that the occurrence of mutations is correlated with a wide range of genomic functions other than the cell cycle-related GO terms.

In addition, to search for the cell cycle-related GO terms related to the occurrence of mutations, the researchers divided sample groups according to the number of mutations and compared GSP distributions. The GO terms (highly significant GO term on mutation, hereinafter HSGO) with a high correlation with mutations were Nos. 3, 18, 2, 20, 39, 15, 32, 55, 33, 4, 75, 35, 7, 69, 29, 22, 28, 74, and 54 (p-value<$5.0 \times 10^{-9}$). The GO terms are arranged in order of decreasing significance of the GSP difference between two groups by comparing the GSP distributions in the sample group with more than 90 mutations and the sample group with fewer than 90 mutations. Even GO term No. 58, which has the lowest correlation with mutations among 78 cell cycle-related GO terms, had a p-value less than 0.05 (p-value=0.045). Therefore, this means that all 78 cell cycle-related GO terms are directly or indirectly related to the occurrence of mutations in breast cancer.

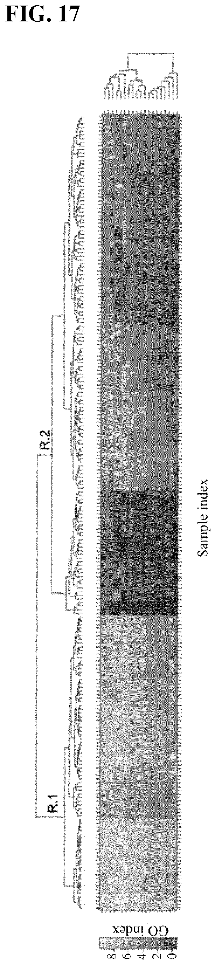

The researchers classified the BRCA samples based on the GSP for the HSGO to accurately identify the correlation between the occurrence of mutations and the cell cycle-related GO terms. FIG. 17 is an example of performing hierarchical clustering of BRCA samples based on the GSP for specific GO terms. FIG. 17 shows the results of performing hierarchical clustering of BRCA samples based on the GSP of specific GO terms. The clustering results hierarchically divide all samples into groups into groups to the left and right of the root. The numbers of mutations (mean$\pm$SD) in the samples belonging to the left (R. 1) and right (R.2) groups were 89.6$\pm$52.2 and 129.7$\pm$58.3, respectively, and there was a significant (p-value=8.63 $\times$ 10$^{-7}$) difference. Furthermore, the researchers performed a linear regression between the logarithm of the number of mutations and the GSP for each GO term in the two sample groups. As the results of the linear regression, in the R. 1 group, no significant (p-value<0.05) GO terms were found, but in the R.2 group, 11 significant GO terms (36, 15, 38, 14, 16, 23, 26, 3, 63, 13 and 54) were observed.

These research results clearly indicate that the GSP may be an indicator for evaluating tumor-like phenotypes for GO term gene sets. That is, the value of the GSP for the GO term gene set for a specific sample (patient) may provide information on the onset of the tumor, the degree of malignancy of the tumor, the target of tumor treatment, etc.

**GO log odds ratio (GLOR)**

As described above, searching for genes involved in the deviation of the genomic system of the cell cycle-related GO term gene set from the normal state in individual tumor patients is of important value in a practical sense. The extent to which each gene is involved in deviation from normal in an individual tumor sample cannot be determined from the expression level of that gene, and its contribution to deviation as a component of the system should be measured.

The gene to be analyzed is called a target gene. In an individual patient's sample, an odds ratio of the sample's SP before and after removing a specific target gene from a genomic system indicates the degree to which the target gene is related to deviation from normal.

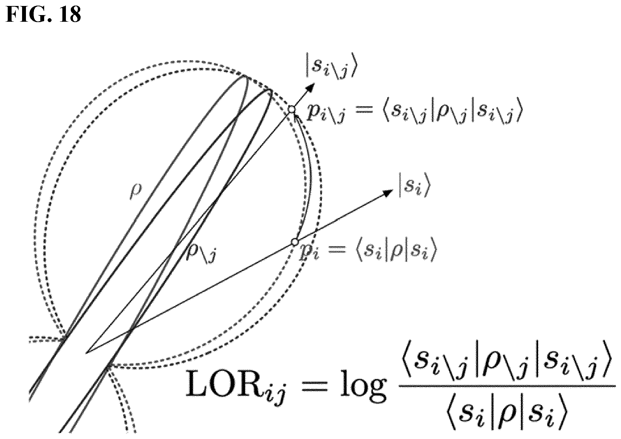

A gene group G composed of n genes forms an n-dimensional gene space $\mathbb{R}^n$ . Within this space, the expression data of G measured in sample i defines the expression vector $|s_i\rangle$. Within this gene space, the density matrix $\rho$ of the gene expression data measured in normal samples defines the emerging traits of the corresponding gene group. FIG. 18 is an example of describing the LOR of a specific gene in an individual sample for the genomic system having a density matrix p. The activity of G in one sample i may be expressed as sample probability $p_i$, as shown in FIG. 18. Meanwhile, the degree to which sample probability $p_{i\backslash j}$ (sample probability when gene j is excluded from G) of sample i deviates from $p_i$ may be known as LOR$_{ij}$ for the sample probability expressed in Equation 11 below. The LOR$_{ij}$ can be consideration the contribution of gene j in sample i to the genomic system composed of gene group G.

$$[\text{Equation 11}]$$

$$\mathrm{LOR}_{ij} = \log \frac{\langle s_{i\backslash j} | \rho_{\backslash j} | s_{i\backslash j} \rangle}{\langle s_i | \rho | s_i \rangle}$$

$p_i$ is the SP for G of sample i, and $p_{i\backslash j}$ is the SP for genes other than gene j in G of sample i. The LOR represents the degree to which each gene in an individual sample is correlated with the disruption of the genomic system in a tumor.

Furthermore, the LOR for the gene group (GO term gene set) related to the GO term among the genes of sample i may be calculated. The LOR targeting the GO term gene set is named GLOR. In this case, the GLOR indicates the degree to which gene j contributes to the GO term gene set. The GLOR may be calculated using Equation 11 above, in which $p_i$ is the SP for G of sample i, and G is the GO term gene set.

The researchers calculated the GLOR for each cell cycle-related GO term gene set in breast cancer tissue (BRCA). The researchers identified the extent to which each gene in individual BRCA samples for GO term No. 6 (cell division) was involved in the deviation of the genomic system of the GO term from the normal state.

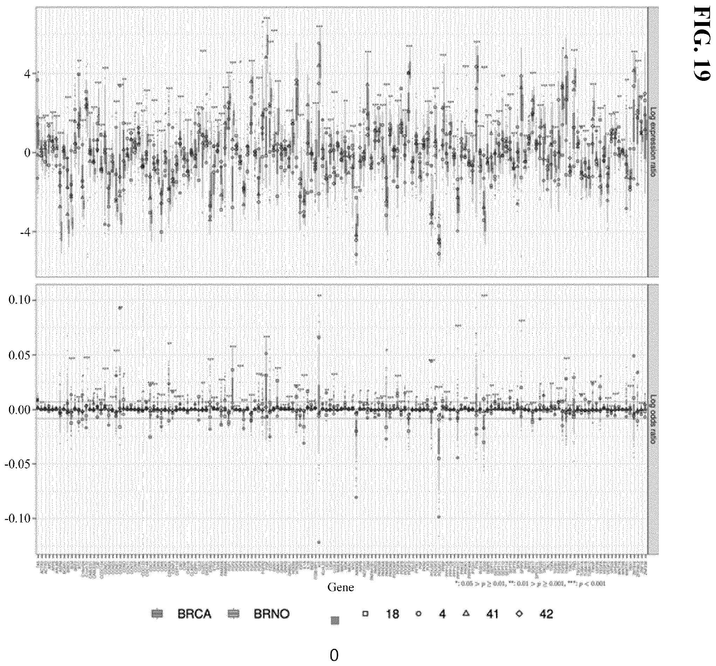

To compare with the results of the GLOR, the researchers calculated LER(log expression to compare with the results of the GLOR, the researchers calculated LER(log expression ratio) for the gene set of the same sample. The LER is gene expression data measured using microarray technology and means an mRNA fluctuation rate of each gene. The LER is data refined through the log2 LOWESS normalization process that uses the mRNA fluctuation rate of each gene in a standard way. Although the distribution of the LER of individual samples may be compared between control and experimental groups in each gene, it is difficult to provide biological meaning. FIG. 19 is an example of comparing the GLOR of each gene with the LER for the genomic system of a specific GO term in BRCA and BRNO samples. FIG. 19A shows the results of calculating the GLOR of each gene for the genomic system of GO term No. 6 in the BRCA and BRNO samples. FIG. 19B shows the LER of the gene belonging to GO term No. 6 in the BRCA and BRNO samples. In FIG. 19A, there were 122 genes out of 163 showing a significant (p<0.05) difference between the LERs of BRCA and BRNO. However, since LER mutations are not related to the GO, they do not show the contribution of genes to the dysfunction of the cellular function of cell division. On the other hand, in FIG. 19B, the GLOR showed significant differences between BRCA and BRNO in 108 genes. There was no significant difference in the LER distribution range of each gene between BRNO and BRCA, with the interquartile range (IQR) of BRCA being 1.67 times that of BRNO on average. In addition, the distribution area also varied greatly depending on the gene. On the other hand, the IQR of the GLOR of BRCA reached an average of 9.93 times that of BRNO. The distribution area of BRNO is also concentrated near 0, and the distribution of the GLOR of BRCA genes shows a pattern of expanding upward and/or downward centered on 0 as a whole. This phenomenon is due to the GLOR of the sample in which the disruption of the genomic system is minimal being within or close to the GLOR distribution area of BRNO. Using these characteristics of the GLOR, genes highly related to GO disruption in tumors were extracted. First, the median of the LOR of individual gene i was calculated, and genes located outside $\mu(\tilde{m}_i) \pm 2SD(\tilde{m}_i)$ were searched for. In the 248 BRCA samples, on average, 6 genes (FGF2, FIGF, NANOG, PDGFD, POU5F1, and SCG2) in GO term No. 6 significantly contributed to the disruption of the genomic system of "cell division" in breast cancer.

Looking at the meaning of the GLOR in individual patients, in the case of BRCA patient No. 41 (p=0.657) with the largest SP, the GLOR was distributed close to BRNO in all genes, and there were no genes with the LOR distributed outside the IQR of BRCA. BRCA patient No. 42 (p=0.630) with slightly smaller SP has a slightly larger deviation from the distribution range of BRNO than patient No. 41, but is still close to the distribution range of BRNO, and there were only two genes, POU5F1 and SCG2, in which the GLOR was distributed outside the IQR. On the other hand, the GLOR of BRCA patient No. 4 (p=0.155) with the smallest SP, was biased toward both extremes, with 49 genes out of 163 distributed outside the IQR. In addition, patient No. 18 (p=0.191) with an SP slightly larger than that of patient No. 4 also showed a tendency to be distributed to be biased toward both extremes, and the number of genes distributed outside the IQR reached 35, although it was fewer than that of patient No. 4.

Since the GLOR calculates the odds ratio of the SP by one gene in the GO term gene set, the LOR of the corresponding gene is bound to be different depending on the GO term. For example, the abnormal spindle-like microcephaly-associated (ASPM) gene is included in GO terms Nos. 6 and 46 (mitotic nuclear division). The ASPM gene does not make a significant contribution to the transformation of the genomic system of GO term No. 6 on average in BRCA, but significantly contributes to the disruption of the genomic system in GO term No. 46.

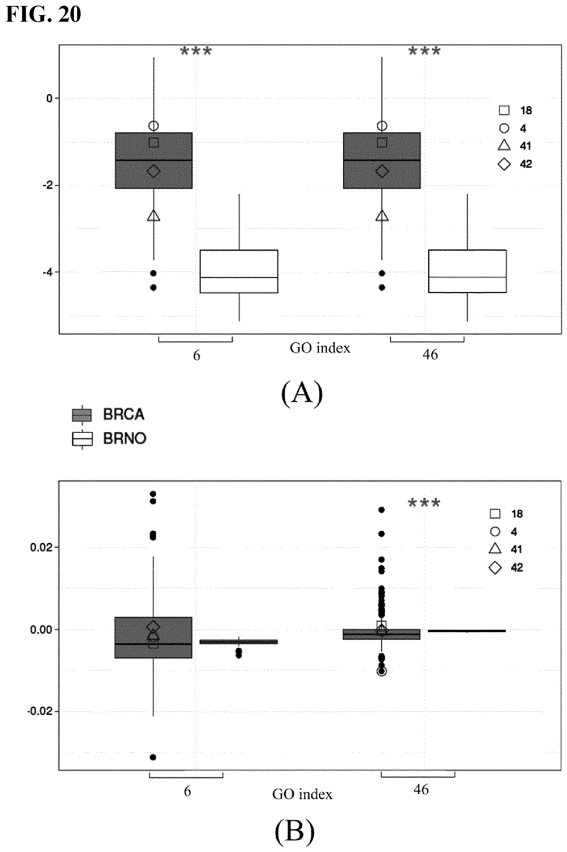

FIG. 20 is an example of the GLOR and the LER of the ASPM gene for the genomic system of cell cycle-related GO term Nos. 6 and 46 in BRCA and BRNO samples. FIG. 20A is an example of the LER, and FIG. 20B is an example of the GLOR. As shown in FIG. 20, the distribution of the LER may not vary depending on the GO term, but the GLOR shows a different distribution in GO term Nos. 6 and 46 for both BRNO and BRCA. Furthermore, the GLOR of individual samples also differs between the two GO terms. These results suggest that the GLOR may quantify the contribution of genes functioning in various biological functions to each relevant genomic system and show the degree of variation of the genomic system in diseases including malignant tumors.

Hereinafter, the process of analyzing samples using the above-described GSP and GLOR is summarized. Meanwhile, the researchers studied cell cycle-related GO terms using breast cancer samples. However, the above-described methodology is not limited to a specific phenotype (disease) or a specific GO term. Therefore, the sample analysis method described below may indicate the degree to which a sample deviates from normal using a set of genes determined by a certain GO term for various phenotypes.

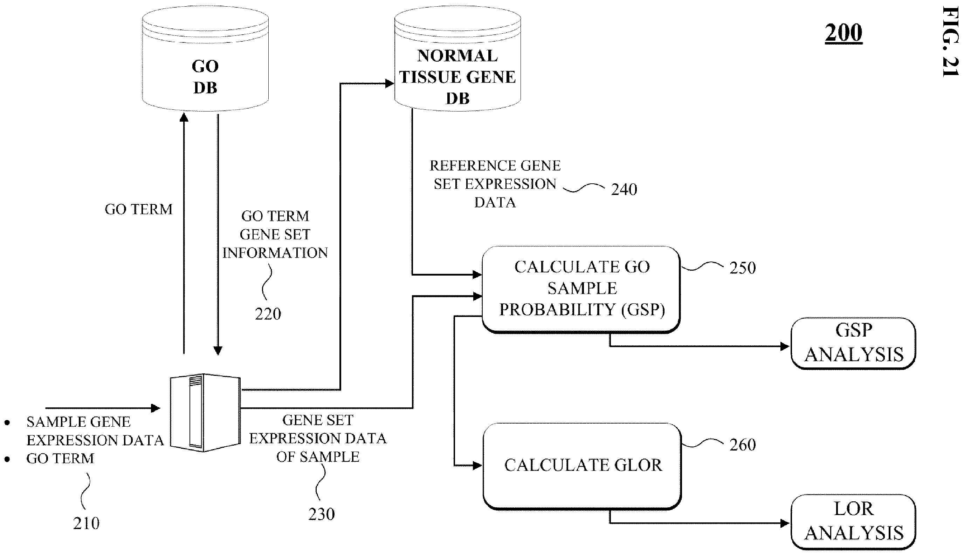

FIG. 21 is an example of a process (220) of calculating an analysis index for a GO-based sample. FIG. 21 shows an example where the analysis device is a server.

The analysis device receives the gene expression data of a sample and a specific GO term (210). The specific GO term may vary depending on the type of disease, treatment target, etc. The GO term may be a plurality of terms.

The analysis device queries a GO database for the received GO term and receives information on the corresponding GO term gene set (220).

The analysis device extracts the expression data of the genes included in the GO term gene set from the gene expression data of the sample (230). Here, the GO term may be a single term or a plurality of terms. In the case of a plurality of GO terms, the extracted genes include genes related to each of the plurality of GO terms.

The analysis device receives the expression data (reference gene set expression data) of the genes included in the GO term gene set from a normal tissue gene database (240). Alternatively, the analysis device receives the gene expression data of normal tissue from the normal tissue gene database and extracts the expression data (reference gene set expression data) of the genes included in the GO term gene set from the received gene expression data (240). Here, the GO term may be a single term or a plurality of terms. In the case of a plurality of GO terms, the extracted genes include genes related to each of the plurality of GO terms.

The analysis device calculates the sample probability (GSP) using the gene expression data of the GO term gene set of the sample and the reference gene set expression data (250). The analysis device may calculate the GSP for each single GO term. Alternatively, the analysis device may calculate the GSP for a plurality of GO terms. The analysis device may derive the analysis results (degree of variation, degree of malignancy of disease, treatment target, etc.) of the sample based on the calculated GSP.

In addition, the analysis device calculates the GLOR using the gene expression data of the GO term gene set of the sample and the reference gene set expression data (260). As described in Equation 10, the analysis device may remove any gene from the GO term gene set of the sample and calculate the GLOR using the SP of the remaining genes and the SP for the entire gene set. The analysis device may calculate the GLOR for each single GO term. Alternatively, the analysis device may also calculate the GLOR for a plurality of GO terms. The analysis device may derive the analysis results (degree of variation, degree of malignancy of disease, treatment target, etc.) of the sample based on the calculated GLOR.

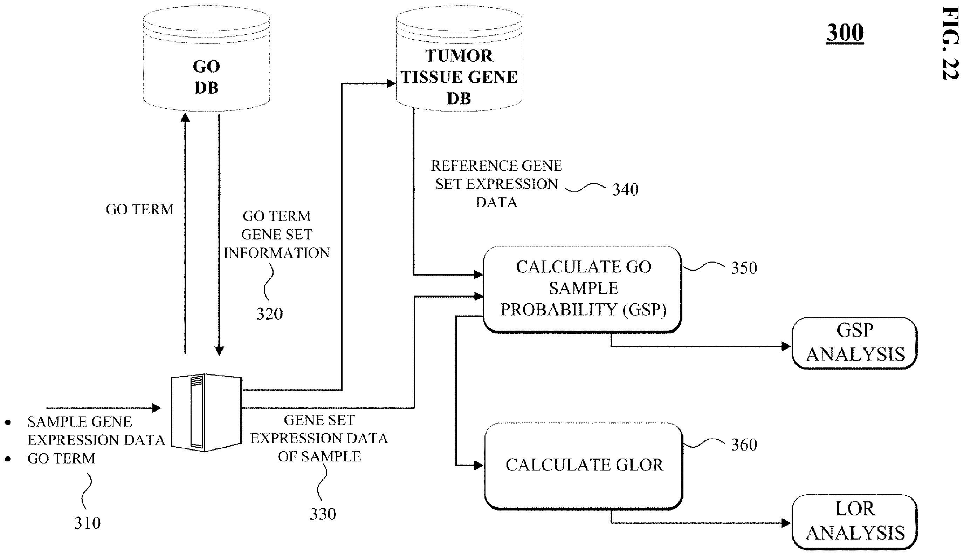

FIG. 22 is another example of a process (300) of calculating an analysis index for a GO-based sample. FIG. 21 shows an example where the analysis device is a server. Unlike Figure 21, in FIG. 22, the reference tissue is not normal tissue but tumor tissue. FIG. 21 is an example where the analysis device calculates the GSP or LOR for a sample in relation to normal tissue. FIG. 22 is an example where the analysis device calculates the GSP or LOR for a sample in relation to tumor tissue. Therefore, the GSP calculated by the analysis device provides information on the degree to which the sample resembles tumor tissue. The LOR calculated by the analysis device represents the degree to which any gene in a sample contributes to the sample tissue having characteristics similar to tumor tissue. Such results may be obtained by using the reference data of tumor tissue instead of normal tissue in the above-described GSP and LOR calculation process.

The analysis device receives the gene expression data of a sample and a specific GO term (310). The specific GO term may vary depending on the type of disease, treatment target, etc. The GO term may be a plurality of terms.

The analysis device queries the GO database for the received GO term and receives information on the corresponding GO term gene set (320).

The analysis device extracts the expression data of the genes included in the GO term gene set from the gene expression data of the sample (330). Here, the GO term may be a single term or a plurality of terms. In the case of a plurality of GO terms, the extracted genes include genes related to each of the plurality of GO terms.

The analysis device receives the expression data (reference gene set expression data) of the genes included in the GO term gene set from a tumor tissue gene database (340). Alternatively, the analysis device receives the gene expression data of tumor tissue from the tumor tissue gene database and extracts the expression data (reference gene set expression data) of the genes included in the GO term gene set from the received gene expression data (340). Here, the GO term may be a single term or a plurality of terms. In the case of a plurality of GO terms, the extracted genes include genes related to each of the plurality of GO terms.

The analysis device calculates the sample probability (GSP) using the gene expression data of the GO term gene set of the sample and the reference gene set expression data (350). The analysis device may calculate the GSP for each single GO term. Alternatively, the analysis device may calculate the GSP for a plurality of GO terms. The analysis device may derive the analysis results (degree of variation, degree of malignancy of disease, treatment target, etc.) of the sample based on the calculated GSP.

In addition, the analysis device calculates the GLOR using the gene expression data of the GO term gene set of the sample and the reference gene set expression data (360). As described in Equation 10, the analysis device may remove any gene from the GO term gene set of the sample and calculate the GLOR using the SP of the remaining genes and the SP for the entire gene set. The analysis device may calculate the GLOR for each single GO term. Alternatively, the analysis device may calculate the GLOR for a plurality of GO terms. The analysis device may derive the analysis results (degree of variation, degree of malignancy of disease, treatment target, etc.) of the sample based on the calculated GLOR.

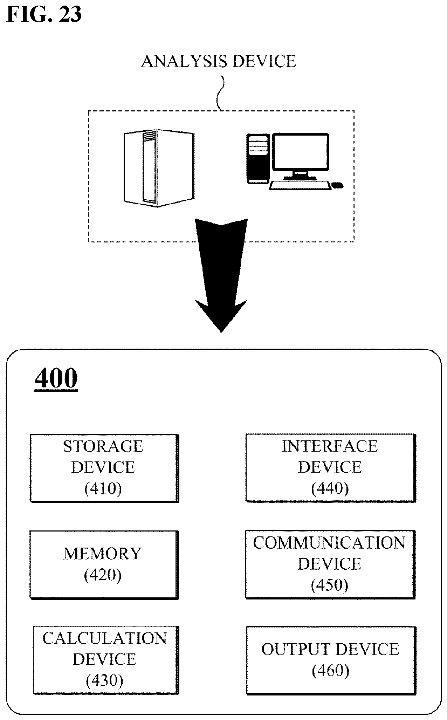

FIG. 23 is an example of an analysis device 400 that analyzes GO-based gene data. The analysis device 400 is a device corresponding to the analysis device 140, 150, or 160 of FIG. 1. The analysis device 400 may be physically implemented in various forms. For example, the analysis device 400 may have the form of a computer device such as a PC, a smart device, a server of a network, and a chipset dedicated to data processing.

The analysis device 400 may include a storage device 410, a memory 420, a calculation device 430, an interface device 440, a communication device 450, and an output device 460.

The storage device 410 may store a program, source code, or the like required for data processing.

The storage device 410 may store the gene expression data of a sample.

The storage device 410 may store the gene expression data (reference gene expression data) of normal tissue or tumor tissue.

The storage device 410 may store analysis results and information obtained by visually processing the analysis results.

The memory 420 may store data and information generated while the analysis device 400 analyzes GO-based gene data.

The interface device 440 is a device that receives certain commands and data from the outside.

The interface device 440 may receive the gene expression data of a sample.

The interface device 440 may receive reference gene expression data.

The interface device 440 may receive the gene expression data of the sample and/or reference gene expression data from a physically connected input device or an external storage device.

The interface device 440 may receive a GO term. The interface device 440 may receive gene set information related to the specific GO term(s).

The communication device 450 refers to a component for receiving and transmitting certain information through a wired or wireless network.

The communication device 450 may receive the gene expression data of a sample from an external object.

The communication device 450 may receive reference gene expression data from the external object.

The communication device 450 may receive a GO term. The communication device 450 may receive gene set information related to the specific GO term(s).

The communication device 450 may transmit the analysis results of the sample to the external object.

The communication device 450 or the interface device 440 is a device that receives certain data or commands from the outside. The communication device 450 or the interface device 440 may be referred to as an input/output device. In addition, considering only the ability to input information, the communication device 450 to the interface device 440 may be called an input device.

The output device 460 is a device that outputs certain information. The output device 460 may output an interface necessary for a data processing process, analysis results, and the like.

The calculation device 430 may regularly preprocess the gene expression data of the sample and/or the reference gene expression data.

The calculation device 430 may extract the expression data of genes belonging to the GO term-related gene set from the gene expression data of the sample based on the information on the GO term-related gene set.

The calculation device 430 may extract the expression data of the genes belonging to the GO term-related gene set from the reference gene expression data based on the information on the GO term-related gene set. Here, the GO term includes at least one term.

The calculation device 430 may calculate the GSP using the gene expression data of the sample and reference gene expression data for the gene set related to the GO term. The GSP calculation process is the same as described above.

The calculation device 430 may calculate the GLOR, which is information on the contribution of any one gene to the degree of variation for the gene set related to the GO term. The GLOR calculation process is the same as described above.

The calculation device 430 may derive the analysis results (degree of variation, degree of malignancy of the disease, treatment target, etc.) of the sample based on the GSP and/or GLOR of the sample.

The calculation device 430 may be a device such as a processor, an AP, or a chip embedded with a program that processes data and performs certain calculations.

In addition, the method of analyzing the genomic data of a sample as described above may be implemented as a program (or application) including an executable algorithm that can be executed on a computer. The program may be stored and provided in a non-transitory computer readable medium.

The non-transitory computer-readable medium is not a medium that stores data for a short period of time, such as a register, a cache, a memory, or the like, but a medium that semi-permanently stores data and is readable by a device. Specifically, various applications or programs described above may be provided by being stored in non-transitory readable media such as a compact disc (CD), a digital video disc (DVD), a hard disk, a Blu-ray disc, a universal serial bus (USB), a memory card, a read-only memory (ROM), a programmable read only memory (PROM), an erasable PROM (EPROM), an electrically EPROM (EEPROM), or a flash memory.

The transitory readable media refer to various RAMs such as a static RAM (SRAM), a dynamic RAM (DRAM), a synchronous DRAM (SDRAM), a double data rate SDRAM (DDR SDRAM), an enhanced SDRAM (ESDRAM), a synclink DRAM (SLDRAM), and a direct rambus RAM (DRRAM).

The technology described above is a new approach to effectively analyze the phenotype of a sample using only gene expression information. The technology described above provides a diagnosis and treatment method for specific diseases by interpreting previously used gene ontology information into information that can be used to treat actual diseases.

The present embodiments and the drawings attached to the present specification merely clearly show some of the technical ideas included in the above-described technology, and therefore, it will be apparent that all modifications and specific embodiments that can be easily inferred by those skilled in the art within the scope of the technical spirit included in the specification and drawings of the above-described technology are included in the scope of the above-described technology.

**Claims**

1. A method of analyzing genetic data based on gene ontology, comprising:

   receiving, by an analysis device, gene expression data of a sample and gene expression data of reference tissue;
   receiving, by the analysis device, information on gene set related to at least one gene ontology term;
   extracting, by the analysis device, first gene expression data for genes belonging to the gene set from among the gene expression data of the sample and second gene expression data for genes belonging to the gene set from among the gene expression data of the reference tissue; and
   calculating, by the analysis device, a degree of variation of the sample based on the reference tissue by calculating entropy for the first gene expression data and the second gene expression data,
   wherein the entropy represents non-uniformity of a probability distribution in a density matrix defining a transcriptional state of the genes belonging to the gene set.

2. The method of claim 1, wherein the analysis device determines the degree of variation by obtaining the density matrix in a gene space using the first gene expression data and obtaining an expression vector using the second gene expression data.

3. The method of claim 1, wherein the degree of variation is calculated by the following Equation,

$$P(s_i|G_\alpha) = \sigma_{i\alpha}^{\top} \rho_\alpha^{(s)} \sigma_{i\alpha},$$

$$\rho_\alpha^{(s)} = \frac{\mathbf{G}_\alpha \mathbf{G}_\alpha^{\top}}{tr(\mathbf{G}_\alpha \mathbf{G}_\alpha^{\top})},$$

$$\sigma_{i\alpha} = \frac{\mathbf{s}_{i\alpha}}{\|\mathbf{s}_{i\alpha}\|}$$

   (Here, $P(s_i|G_\alpha)$ denotes a sample probability, $G_\alpha$ denotes an expression matrix of a gene set included in the gene set $\alpha$ in reference genome expression data, and $s_{i\alpha}$ denotes a gene expression vector included in $\alpha$ in sample expression data $s_i$).

4. The method of claim 1, further comprising comparing, by the analysis device, the degree of variation between the first gene expression data and remaining gene expression data excluding specific genes from the first gene expression data to determine a degree of contribution of the specific gene to the degree of variation.

5. The method of claim 4, wherein the analysis device calculates a log odds ratio (LOR) based on the degree of variation of the first gene expression data and the degree of variation of the remaining gene expression data to determine the degree of contribution.

6. A device for analyzing genetic data based on gene ontology, comprising:

   an input device that receives information on gene set related to at least one gene ontology term;
   a storage device that stores gene expression data of a sample and gene expression data of reference tissue; and
   a calculation device that extracts first gene expression data for genes belonging to the gene set from among the gene expression data of the sample and second gene expression data for genes belonging to the gene set from among the gene expression data of the reference tissue, and calculates a degree of variation of the sample based on the reference tissue by calculating entropy for the first gene expression data and the second gene expression data,
   wherein the entropy represents non-uniformity of a probability distribution in a density matrix defining a transcriptional state of the genes belonging to the gene set.

7. The device of claim 6, wherein the gene set information is acquired by querying the gene ontology term in a gene ontology database.

8. The device of claim 6, wherein the calculation device determines the degree of variation by obtaining the density matrix in a gene space using the first gene expression data and obtaining an expression vector using the second gene expression data.

9. The device of claim 6, wherein the degree of variation is calculated by the following Equation,

$$P(s_i|G_\alpha) = \sigma_{i\alpha}^\top \rho_\alpha^{(s)} \sigma_{i\alpha},$$

$$\rho_\alpha^{(s)} = \frac{G_\alpha G_\alpha^\top}{tr(G_\alpha G_\alpha^\top)},$$

$$\sigma_{i\alpha} = \frac{s_{i\alpha}}{\|s_{i\alpha}\|}$$

(Here, $P(s_i|G_\alpha)$ denotes a sample probability, $G_\alpha$ denotes an expression matrix of a gene set included in the gene set $\alpha$ in reference genome expression data, and $s_{i\alpha}$ denotes a gene expression vector included in $\alpha$ in sample expression data $s_i$).

10. The device of claim 6, wherein the calculation device calculates a log odds ratio (LOR) based on the degree of variation of the first gene expression data and the degree of variation of the remaining gene expression data excluding a specific gene from the first gene expression data to determine a degree of contribution of the specific gene to the degree of variation.

## FIG. 1

**FIG. 2**

$|1\rangle$ on

$|0\rangle$ off

(A)

(B)

**FIG. 3**

(A)

(B)

**FIG. 4**

**FIG. 5**

FIG. 6

**FIG. 7**

**FIG. 8**

**FIG. 9**

**FIG. 10**

FIG. 11

**FIG. 12**

**FIG. 13**

**FIG. 14**

**FIG. 15**

**FIG. 16**

FIG. 17

**FIG. 18**

$$\mathrm{LOR}_{ij} = \log \frac{\langle s_{i\backslash j}|\rho_{\backslash j}|s_{i\backslash j}\rangle}{\langle s_i|\rho|s_i\rangle}$$

## FIG. 19

EP 4 456 075 A1

**FIG. 20**

(A)

(B)

FIG. 21

**200**

EP 4 456 075 A1

FIG. 22

# FIG. 23

ANALYSIS DEVICE

**400**

| | |
|---|---|
| STORAGE DEVICE (410) | INTERFACE DEVICE (440) |
| MEMORY (420) | COMMUNICATION DEVICE (450) |
| CALCULATION DEVICE (430) | OUTPUT DEVICE (460) |

## INTERNATIONAL SEARCH REPORT

| International application No. |
| --- |
| **PCT/KR2022/020687** |

**A. CLASSIFICATION OF SUBJECT MATTER**

**G16B 25/10**(2019.01)i; **G16B 50/10**(2019.01)i; **G16B 20/20**(2019.01)i; **G16B 45/00**(2019.01)i

According to International Patent Classification (IPC) or to both national classification and IPC

**B. FIELDS SEARCHED**

Minimum documentation searched (classification system followed by classification symbols)

G16B 25/10(2019.01); A61B 5/00(2006.01); C12M 1/00(2006.01); C12M 1/34(2006.01); C12Q 1/68(2006.01); G06F 19/22(2011.01); G06F 19/24(2011.01); G06F 19/28(2011.01); G16B 20/00(2019.01); G16B 5/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 유전자 발현 데이터(gene expression data), 유전자 온톨로지 텀(gene ontology term), 엔트로피(entropy), 전사(transcription), 밀도 행렬(density matrix), 발현 벡터(expression vector), 변이(variation)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2019-093695 A1 (INDUSTRY-UNIVERSITY COOPERATION FOUNDATION HANYANG UNIVERSITY) 16 May 2019 (2019-05-16)<br>See claims 1-24; and paragraph [0006]. | 1-10 |
| A | KR 10-2008-0086332 A (ELECTRONICS AND TELECOMMUNICATIONS RESEARCH INSTITUTE) 25 September 2008 (2008-09-25)<br>See claims 1-12. | 1-10 |
| A | US 2018-0165413 A1 (SOOCHOW UNIVERSITY) 14 June 2018 (2018-06-14)<br>See claims 1-10. | 1-10 |
| A | WO 2019-131785 A1 (KYOTO UNIVERSITY) 04 July 2019 (2019-07-04)<br>See claims 1-9. | 1-10 |
| A | KR 10-2020-0131750 A (IUCF-HYU (INDUSTRY-UNIVERSITY COOPERATION FOUNDATION HANYANG UNIVERSITY)) 24 November 2020 (2020-11-24)<br>See claims 1-14. | 1-10 |

☐ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **07 April 2023** | **07 April 2023** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2022/020687**

| Patent document cited in search report | | | Publication date (day/month/year) | Patent family member(s) | | | Publication date (day/month/year) |
|---|---|---|---|---|---|---|---|
| WO | 2019-093695 | A1 | 16 May 2019 | KR | 10-2019-0054386 | A | 22 May 2019 |
| | | | | KR | 10-2019-0095704 | A | 16 August 2019 |
| | | | | KR | 10-2019-0143043 | A | 30 December 2019 |
| | | | | KR | 10-2051710 | B1 | 04 December 2019 |
| | | | | KR | 10-2110262 | B1 | 13 May 2020 |
| | | | | KR | 10-2137029 | B1 | 23 July 2020 |
| | | | | US | 2020-0286582 | A1 | 10 September 2020 |
| | | | | US | 2020-0372972 | A1 | 26 November 2020 |
| KR | 10-2008-0086332 | A | 25 September 2008 | KR | 10-0964181 | B1 | 17 June 2010 |
| | | | | US | 2009-0112480 | A1 | 30 April 2009 |
| US | 2018-0165413 | A1 | 14 June 2018 | CN | 105825081 | A | 03 August 2016 |
| | | | | CN | 105825081 | B | 14 September 2018 |
| | | | | EP | 3299976 | A1 | 28 March 2018 |
| | | | | WO | 2017-181665 | A1 | 26 October 2017 |
| WO | 2019-131785 | A1 | 04 July 2019 | | None | | |
| KR | 10-2020-0131750 | A | 24 November 2020 | EP | 3970606 | A1 | 23 March 2022 |
| | | | | KR | 10-2346561 | B1 | 03 January 2022 |
| | | | | US | 2022-0215268 | A1 | 07 July 2022 |
| | | | | WO | 2020-231184 | A1 | 19 November 2020 |

Form PCT/ISA/210 (patent family annex) (July 2022)